# EUROPÄISCHE PATENTANMELDUNG

(11) **EP 4 644 521 A1**
(43) Veröffentlichungstag der Anmeldung: **05.11.2025**
(21) Anmeldenummer: 24172951.6
(22) Anmeldetag: 29.04.2024
(51) Int. Cl.: C12M 1/00, B01L 1/02, C12M 3/06

(54) **LABORGERÄT MIT SCHWENKTÜRE**

(71) Anmelder: Eppendorf SE, 22339 Hamburg (DE)
(72) Erfinder: Fitzer, Jan, Ammersbek (DE); Stöhrer, Frederic, Itzstedt (DE)
(74) Vertreter: Wallinger Ricker Schlotter Tostmann

(57) **Zusammenfassung**

Die Erfindung betrifft einer Laborschüttler zum Schütteln von in Probengefäßen gelagerten Laborproben in einer Kammer, der eine Antriebskomponenten tragende Schubladeneinrichtung aufweist, die zu Wartungszwecken aus einem Schubladenraum des Laborschüttlers ausfahrbar ist.

## Beschreibung

Die Erfindung betrifft Laborschüttler für das Schütteln von in Probengefäßen gelagerten Laborproben, insbesondere von Mikroorganismen in Suspension, vorzugsweise Säugerzellen in Suspension.

Temperaturgesteuerte Laborschüttlerwerden in biologischen, medizinischen und pharmazeutischen Laboren für die Kultivierung von Zellen, Bakterien, Hefe und anderen Organismen in Suspension verwendet. Sie sind z. B. für die Produktion rekombinanter DNA, die Proteinexpression oder das Screening von Kulturen unerlässlich. Da es sich bei einem Laborschüttler in erster Linie um ein gemeinsam genutztes Gerät handelt, das rund um die Uhr bei hohen Drehzahlen und variabler Belastung läuft, muss er langlebig und zuverlässig sein.

Die für einen Benutzer relevanten Kenngrößen für einen Laborschüttler, insbesondere einen Inkubationsschüttler, sind zunächst ein bestimmte Zieltemperatur im Probenlagerraum, eine bestimmte Geschwindigkeit und dazu eine bestimmte Last-Tragfähigkeit der Schüttler-Plattform sowie eine Regelmöglichkeit für die CO2-Konzentration und die Luftfeuchte einer Inkubationsatmosphäre. Die meisten Anwendungen erfordern die Verwendung unterschiedlicher Gefäßgrößen und -typen: von Platten für das erste Screening über konische Gefäße für Vorkulturen bis hin zu großen Kolben für die Plasmidproduktion oder Proteinexpression. Der ständige Bedarf an höheren Produktausbeuten führte zur Erfindung neuer Kolbentypen, die eine bessere Belüftung als Standard-Schüttelkolben bieten. Dadurch kann das typische Füllvolumen um bis zu 40 % erhöht werden, was zu einem höheren Gewicht auf der Plattform führt. Höhere Drehzahlen über 250 U/min sind für Anwendungen mit z. B. E. coli üblich, um eine signifikante Erhöhung der Zelldichte zu erreichen. Beim Schütteln von Probenplatten zum Screening ist es wichtig, eine vollständige Durchmischung zu erreichen und eine Sedimentation der Zellen zu verhindern. Die Anforderungen an Laborschüttler sind deshalb umfangreich, und beinhalten neben der Dauerbelastbarkeit eine ausreichende Versatilität, um insbesondere alle Arten von Plattformkonfigurationen, Lasten und auch hohe Drehzahlen zu bewältigen. Gefordert sind Langlebigkeit und Robustheit für einen zuverlässigen Betrieb über Jahre hinweg.

Als Orbitalschüttler kann ein Laborschüttler bezeichnet werden, bei dem eine Plattform in einer elliptischen oder kreisförmigen Bahn bewegt wird, wobei die Bewegung durch einen Exzenter gesteuert wird. Im Allgemeinen werden Bechergläser, Kolben und andere Gefäße an der Oberseite der Plattform befestigt, so dass die darin enthaltene Flüssigkeit an den inneren Seitenwänden des Gefäßes umhergewirbelt wird, um die Durchmischung zu erhöhen und die Wechselwirkung oder den Austausch zwischen der Flüssigkeit und der lokalen gasförmigen Umgebung zu verbessern.

Als Orbitalschüttler bezeichnet man ferner insbesondere Laborschüttler, die eine Plattform so bewegen, dass sich integral alle Punkte auf der Plattform in einer gemeinsamen, ebenen, orbitalen Bahn bewegen, als Überlagerung zweier Translationen und in der Amplitude durch einen Exzenter definiert. Eine Bewegung in einer gemeinsamen, ebenen, orbitalen Bahn meint insbesondere, dass alle Punkte auf der Plattform des Orbitalschüttlers sich in einer elliptischen oder kreisförmigen Bahn bewegen, wobei die Bahn in einer Ebene liegt. Eine "Überlagerung zweier Translationen" bezieht sich insbesondere darauf, dass die Bewegung der Plattform des Schüttlers als Kombination zweier gerader Bewegungen in unterschiedlichen Richtungen betrachtet werden kann. Diese Überlagerung führt zur elliptischen oder kreisförmigen Bahn. Ein Exzenter ist eine Vorrichtung, die dazu dient, eine translatorische Bewegung in eine rotatorische Bewegung umzuwandeln. In diesem Fall definiert der Exzenter die Amplitude der Bewegung, also die maximale Auslenkung der Plattform. Ein Exzenter in der Mechanik ist eine Steuerungsscheibe, die auf einer Welle befestigt ist und deren Mittelpunkt sich außerhalb der Achse der Welle befindet.

Solche Laborschüttler weisen eine Kammer zur Aufnahme der zu temperierenden Laborproben auf, diese Kammer ist in der Regel innerhalb eines Gehäuses angeordnet. Der Zugang zur Kammer, bei dem der Benutzer die Proben im Gehäuseinneren, insbesondere in der Kammer, lagert und wieder entnimmt, erfolgt in der Regel über eine Gehäuseöffnung, die mittels einer Gehäusetüre verschließbar ist. In weiteren Ausgestaltungen verfügt die Kammer auch über eine Gaszufuhr. Diese Arten von Geräten erlauben die Kultivierung von Zellen in einer CO₂ Atmosphäre. Sie werden Inkubationsschüttler genannt. Ein bekannter Laborschüttler ist der Innova^{®} S44i, erhältlich von der Eppendorf SE, Hamburg, Deutschland.

Kontamination ist eines der größten Sicherheitsrisiken bei der Arbeit mit Laborgeräten zur Behandlung von Laborproben in einer Kammer. In solchen Laborgeräten, insbesondere bei CO2 Inkubatoren mit Schüttelantrieb, ist die Reinigbarkeit eine extrem wichtige Kundenanforderung. Hierbei steht die Kammer im Fokus, in welche die Proben vom Anwender eingebracht werden und dort über eine lange Zeit inkubiert werden. Die Kammer muss zum einen für den Anwender erreichbar und zum anderen von der Umwelt abgeschottet sein, um ein spezielles Klima unabhängig von den Umweltbedingungen zu erzeugen (Temperatur, CO2, Feuchte). Eine Tür ist somit notwendig für ein solches System.

Bekannte Laborgeräte weisen Schwenktüren auf, die über eine Schwenkmechanik bzw. Schwenkarme zum Öffnen und Schließen der Kammeröffnung am Laborgerät montiert sind. Diese bekannten Schwenkmechaniken haben den Nachteil, dass sie innerhalb der Kammer verortet sind. Denn das führt dazu, dass diese Teile regelmäßig gereinigt werden müssen. Die Schwenkmechanik ist meist durch eine Umhausung abgedeckt. Die oftmals komplexe Form und Anordnung bzw. die schwer zugänglichen Konturen dieser Komponenten führen gemäß den der Erfindung zugrunde liegenden Beobachtungen dazu, dass Verunreinigungen und Kontaminationen in diesem Bereich der Kammer vom Anwender schlecht oder gar nicht erreicht und somit nicht gut entfernt werden können. Zudem muss ein Benutzer oder Servicetechniker bei dieser Anordnung in die Kammer eingreifen, um eine Reinigung oder Wartung der Antriebskomponenten durchzuführen, was das Risiko einer zusätzlichen Kontamination erhöht. Zudem muss ein Benutzer oder Servicetechniker bei dieser Anordnung in die Kammer eingreifen, um eine Reinigung oder Wartung der Antriebskomponenten durchzuführen, was das Risiko einer zusätzlichen Kontamination erhöht. Durch das Ausführen der Wartungsarbeiten in der Kammer setzt sich der Servicetechniker selber auch einem Kontaminations- / Infektionsrisiko aus, da die Kammer selber kontaminiert sein kann. Bewegliche Teile zu reinigen ist aufwendig und birgt die Gefahr der Verletzung.

Der vorliegenden Erfindung liegt deshalb die Aufgabe zugrunde, ein Laborgerät mit einer Kammer zur Behandlung von Laborproben bereitzustellen, das einfach und sicher zu reinigen und zu warten ist.

Die Erfindung löst diese Aufgabe durch das Laborgerät gemäß Anspruch 1. Bevorzugte Ausgestaltungen der Erfindung sind Gegenstand der Unteransprüche und ergeben sich aus der vorliegenden Beschreibung der Erfindung und den Figuren.

Durch die außerhalb der Kammer vorgesehene Anordnung der Schwenktüreinrichtung erfordert die Reinigung des Laborgeräts einen geringeren Aufwand, als das bei Geräten des Stands der Technik mit innerhalb der Kammer gelegenen Teilen der Türkinematik bislang der Fall ist. Eine optimierte Reinigbarkeit führt zu weniger Kontaminationen innerhalb des Probenraums, was wiederum die Wiederholung von in der Kammer ausgeführten Experimenten oftmals obsolet werden lässt und zum anderen (bei nicht detektierten Kontaminationen) die Reproduzierbarkeit von Ergebnissen unterstützt.

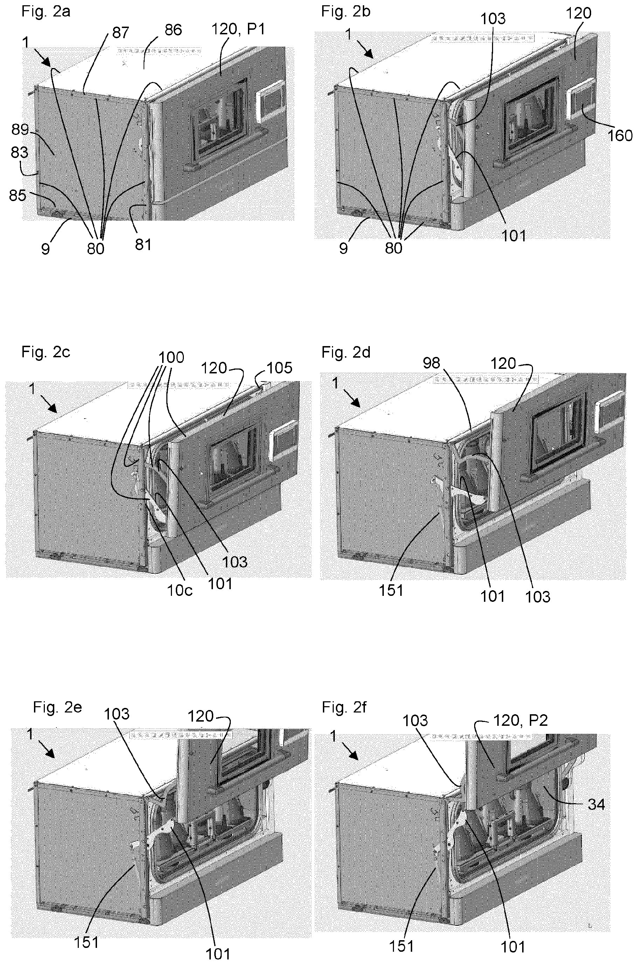

Zum vollständigen Öffnen des Türblattelements zieht der Benutzer den Handgriff weiter nach vorne (Figuren 2c bis 2e). Dadurch wird die Schwenktüre, geführt durch die Schwenktüreinrichtung, nach oben bewegt.

Die Schwenktüreinrichtung ist vorzugsweise dazu eingerichtet, die Bewegung des Öffnens des Türblattelements maschinell zu unterstützen. Vorzugsweise weist die Schwenktüreinrichtung ein Federsystem auf. Die im Federsystem durch Spannung einer Feder speicherbare Energie ist dazu nutzbar, das Nach-oben-Schwenken des Türblattelements zu unterstützen. Dadurch sinkt die zum Öffnen des Türblattelements erforderliche Kraft, was insbesondere bei manuell auszulenkendem Türblattelement für den Benutzer komfortabel ist, was aber auch vorteilhaft für die Gestaltung eines optional motorgetriebenen Öffnungsmechanismus des Türblattelements ist. Die Tür hat in optionalen bevorzugten Ausgestaltungen ein erhöhtes Eigengewicht, wenn sie die dazu eingerichtet ist, die Temperierung der Türe als Kammerfrontwand zu realisieren, oder wenn sie ein Fenster aufweist, um hindurchsehen zu können, was insbesondere separat beheizbar ist.

Bevorzugt ist generell, dass das Federsystem mindestens eine Feder beinhaltet. Diese kann eine Schraubenfeder, insbesondere eine Druckfeder oder eine Zugfeder sein, oder eine Gasfeder, insbesondere eine Gasdruckfeder oder eine Gaszugfeder sein. Die Federkraft einer Feder kann insbesondere zwischen 200 N und 800 N betragen, vorzugsweis zwischen 250 N und 400 N, vorzugsweise zwischen 280 N und 350 N, vorzugsweise 320 N. Es sind insbesondere auch zwei Federn vorgesehen, so dass deren Gesamtkraft die genannten Angaben verdoppelt.

Gaszugfedern nutzen, ebenso wie Gasdruckfedern, ein Gas, das im komprimierten Zustand Energie speichert, die unter Expansion des Gases abgerufen wird und die die manuelle Tätigkeit (hier: Öffnen der Schwenktüre) unterstützt. Gaszugfedern arbeiten mit einer anderen Anordnung als Gasdruckfedern, d.h. durch den Gasdruck im Zylinder wird bei Gaszugfedern die Kolbenstange nach innen in ein Druckrohr gezogen. Eine Gaszugfeder ist eine geschlossene und wartungsfreie Komponente, die aus einem Druckrohr, einer Kolbenstange mit Kolben und einer Dichtung an der Kolbenstangenführung besteht, um Gasverlust zu verhindern. Sie wird mit komprimiertem Gas, insbesondere Stickstoff, gefüllt, um die Federkraft bereitzustellen. Im unbelasteten Zustand ist die Kolbenstange immer eingefahren. Wenn die Kolbenstange herausgezogen wird, verringert sich das Volumen im Zylinder, was zu einer Kompression des Gases führt und somit zu einem Anstieg der Federkraft.

Vorzugsweise wird die Kammer begrenzt durch die erste Seitenwand, die zweite Seitenwand, eine Kammerbodenwand, eine Kammerrückwand und eine Kammerdeckenwand, wobei die Kammerbodenwand, und insbesondere auch die Kammerdeckenwand, im bestimmungsgemäßen Gebrauch des Laborgeräts horizontal angeordnet ist.

Vorzugsweise wird die Kammeröffnung begrenzt durch einen ersten Seitenrand und einen, diesem gegenüberliegend, zweiten Seitenrand, sowie einen Unterrand und, diesem gegenüberliegend, einen Oberrand, wobei der erste und zweite Seitenrand vorzugsweise senkrecht zu dem Oberrand und dem Unterrand verlaufen, wobei vorzugsweise im bestimmungsgemäßen Gebrauch des Laborgeräts der Unterrand, und insbesondere auch der Oberrand, horizontal angeordnet ist.

Vorzugsweise sind der mindestens eine erste Schwenkarm und der mindestens eine zweite Schwenkarm einander gegenüberliegend und parallel zueinander angeordnet und insbesondere parallel zu der ersten Seitenwand und der zweiten Seitenwand angeordnet. Vorzugsweise sind ein erster Schwenkarm und ein zweiter Schwenkarm fest miteinander verbunden, insbesondere unbeweglich zueinander angeordnet, wobei der erste Schwenkarm und der zweite Schwenkarm vorzugsweise durch eine Kopplungsstange fest miteinander verbunden sind. Die Kopplungsstange ist in der Position P1 des Türblattelements vorzugsweise zwischen einer Frontwand des Laborgeräts und einer Innenseite des Türblattelements angeordnet, insbesondere außerhalb der Kammer.

Vorzugsweise weist das Laborgerät eine Basis auf, und damit verbunden, die Trägereinrichtung, die insbesondere eine Rahmeneinrichtung ist. Die Basis und die damit verbundene Trägereinrichtung tragen die weiteren Bestandteile des Laborgeräts.

Vorzugsweise weist die Trägereinrichtung ein erstes Trägerteil mit einem ersten Schwenklager auf, an dem der mindestens eine erste Schwenkarm schwenkbar gelagert ist, und vorzugsweise weist die Trägereinrichtung ein dem ersten Trägerteil gegenüberliegendes zweites Trägerteil mit einem zweiten Schwenklager auf, an dem der mindestens eine zweite Schwenkarm schwenkbar gelagert ist. Dadurch lässt sich eine sichere Lagerung auch eines schweren Türblattelements erreichen. Es ist beispielsweise bevorzugt, dass das Türblattelement ein aus Glas bestehendes oder Glas aufweisendes Sichtfenster aufweist, welches in der ersten Position (P1) den Blick in den Kammerinnenraum ermöglicht. In einer thermisch isolierenden Anordnung eines Glasfensters, insbesondere eines doppel- oder mehrwandigen Glasfensters, ist die Masse des Türblattelements meist größer als im Fall ohne Sichtfenster.

Vorzugsweise ist der maximale Abstand der ersten Seitenwand und der zweite Seitenwand der Kammer geringer als der maximale Abstand des ersten Schwenklagers und des zweiten Schwenklagers. Dies begünstigt die Anordnung der Schwenkarme außerhalb der Kammer.

Die Schwenktüreinrichtung beinhaltet vorzugsweise, insbesondere benachbart dem ersten Seitenrand der Kammeröffnung angeordnet, einen ersten, unteren Schwenkarm und einen ersten, oberen Schwenkarm. Die Schwenktüreinrichtung beinhaltet vorzugsweise, insbesondere benachbart dem zweiten Seitenrand der Kammeröffnung angeordnet, einen zweiten, unteren Schwenkarm und einen zweiten, oberen Schwenkarm. Die ersten Schwenkarme liegen vorzugsweise jeweils den zweiten Schwenkarmen gegenüber und sind vorzugsweise um eine Distanz voneinander beabstandet, welche vorzugsweise größer ist als die maximale Breite des Kammerinnenraums oder der Kammer und welche vorzugsweise kleiner ist als die Gesamtbreite des Laborgeräts.

Das Laborgerät weist vorzugsweise eine erste, schlitzförmige Aussparung auf, in welche der mindestens eine erste Schwenkarm eingreift. Das Laborgerät weist vorzugsweise eine zweite schlitzförmige Aussparung auf, in welche der mindestens eine zweite Schwenkarm eingreift. Die Aussparung mündet vorzugsweise jeweils in einer Öffnung einer Frontwand des Laborgeräts.

Ein erster unterer Schwenkarm und ein erster oberer Schwenkarm sind vorzugsweise schwenkbar an der Trägereinrichtung angeordnet, dass sie während ihrer jeweiligen Schwenkbewegung stets koplanar sind, also in derselben ersten Ebene liegen. Dadurch sind die ersten Schwenkarme kompakt angeordnet. Ein zweiter unterer Schwenkarm und ein zweiter oberer Schwenkarm sind vorzugsweise schwenkbar an der Trägereinrichtung angeordnet, dass sie während ihrer jeweiligen Schwenkbewegung stets koplanar sind, also in derselben zweiten Ebene liegen. Dadurch sind die zweiten Schwenkarme kompakt angeordnet. Die erste und die zweite Ebene sind parallel und zueinander beabstandet.

Vorzugsweise weist die Kammer eine erste Seitenwand und, dieser gegenüberliegend, eine zweite Seitenwand auf, wobei der mindestens eine erste Schwenkarm und der mindestens eine zweite Schwenkarm einander gegenüberliegend und parallel zueinander angeordnet sind und insbesondere parallel zu der ersten Seitenwand und der zweiten Seitenwand angeordnet sind.

Vorzugsweise weist das Laborgerät eine Basis auf, und damit verbunden, die Trägereinrichtung , insbesondere eine Rahmeneinrichtung aufweist, welche Bestandteile des Laborgeräts tragen, wobei die Trägereinrichtung ein erstes Trägerteil mit einem ersten Schwenklager aufweist, an dem der mindestens eine erste Schwenkarm schwenkbar gelagert ist, und die Trägereinrichtung ein dem ersten Trägerteil gegenüberliegendes zweites Trägerteil mit einem zweiten Schwenklager aufweist, aufweist, an dem der mindestens eine zweite Schwenkarm schwenkbar gelagert ist.

Vorzugsweise weist die Trägereinrichtung eine erste Rahmenseitenwand auf, wobei der mindestens eine erste Schwenkarm an einer der Kammer abgewandten Außenseite der Seitenwand angeordnet ist, und wobei die Trägereinrichtung eine zweite Rahmenseitenwand aufweist, und wobei der mindestens eine zweite Schwenkarm an einer der Kammer abgewandten Außenseite der Rahmenseitenwand angeordnet ist, wobei insbesondere die erste Rahmenseitenwand das erste Trägerteil ist und insbesondere die zweite Rahmenseitenwand das zweite Trägerteil ist. Der Zwischenraum zwischen einer ersten Kammerseitenwand und der ersten Rahmenseitenwand der Trägereinrichtung ist vorzugsweise mit einem thermischen Isolationsmaterial gefüllt, und der Zwischenraum zwischen einer zweiten Kammerseitenwand und der zweiten Rahmenseitenwand der Trägereinrichtung ist vorzugsweise mit einem thermischen Isolationsmaterial gefüllt.

Vorzugsweise weist das Laborgerät, insbesondere dessen Schwenktüreinrichtung, ein Federsystem auf, welches das Öffnen des Türblattelements unterstützt, wobei das Federsystem insbesondere durch das Schließen des Türblattelements spannbar ist. Vorzugsweise weist das Laborgerät mindestens ein erstes Federelement auf, insbesondere eine Gasfeder, das an seinem ersten Ende mit einem ersten Schwenkarm und mit seinem zweiten Ende mit der Trägereinrichtung verbunden ist, und das mindestens ein zweites Federelement aufweist, insbesondere eine Gasfeder, das an seinem ersten Ende mit einem zweiten Schwenkarm und mit seinem zweiten Ende mit der Trägereinrichtung verbunden ist.

Vorzugsweise ist ein erster Schwenkarm mittels einem ersten Lagerelement an der Trägereinrichtung um eine erste Schwenkachse schwenkbar gelagert ist, mittels einem zweiten Lagerelement an dem Türblattelement um eine zweite Schwenkachse schwenkbar gelagert, und mittels einem dritten Lagerelement an einem Federelement um eine dritte Schwenkachse schwenkbar gelagert, wobei die erste, zweite und dritte Schwenkachse parallel zueinander verlaufen, und

wobei insbesondere ein zweiter Schwenkarm mittels einem ersten Lagerelement an der Trägereinrichtung um eine erste Schwenkachse schwenkbar gelagert ist, mittels einem zweiten Lagerelement an dem Türblattelement um eine zweite Schwenkachse schwenkbar gelagert ist, und mittels einem dritten Lagerelement an einem Federelement um eine dritte Schwenkachse schwenkbar gelagert ist, wobei die erste, zweite und dritte Schwenkachse parallel zueinander verlaufen.

Ein Lagerelement kann ein Gleitlager oder Wälzlager aufweisen. Es kann ein Schwenklager oder ein Kugelgelenk sein.

Vorzugsweise ist der Abstand zwischen dem ersten Lagerelement und dem zweiten Lagerelement größer, insbesondere 2 bis 4 mal größer ist, als der Abstand zwischen dem ersten Lagerelement und dem dritten Lagerelement.

Vorzugsweise weist der erste Schwenkarm ein erstes Gleitelement auf, das so angeordnet ist, dass es während der Schwenkbewegung des Türblattelements zwischen der ersten Position (P1) und der zweiten Position (P2) im Kontakt mit der ersten Seitenwand an dessen der Kammer abgewandten Außenseite entlang gleitet, und

Insbesondere weist der zweite Schwenkarm ein zweites Gleitelement auf, das so angeordnet ist, dass es während der Schwenkbewegung des Türblattelements zwischen der ersten Position (P1) und der zweiten Position (P2) im Kontakt mit der zweiten Seitenwand an dessen der Kammer abgewandten Außenseite entlang gleitet. Vorzugsweise weist der erste Schwenkarm ein erstes plattenförmiges Bauteil auf, das mit dem ersten Schwenkarm fest verbunden ist und das dieses erste Gleitelement (101b) trägt, und wobei insbesondere der zweite Schwenkarm ein zweites plattenförmiges Bauteil aufweist, das mit dem zweiten Schwenkarm fest verbunden ist und das dieses zweite Gleitelement trägt,

wobei sich das erste plattenförmige Bauteil insbesondere entlang der Länge des ersten Schwenkarms erstreckt, und wobei sich das zweite plattenförmige Bauteil insbesondere entlang der Länge des zweiten Schwenkarms erstreckt.

Vorzugsweise ist in einer planaren Frontwand des Laborgeräts im Abstand zu der Kammer eine schlitzförmige Aussparung vorgesehen, die sich senkrecht zu der Frontwand in das Innere des Laborgeräts erstreckt, und in der der mindestens eine erste Schwenkarm in der ersten Position (P1) des Türblattelements angeordnet ist, und
wobei insbesondere in der planaren Frontwand des Laborgeräts im Abstand zu der Kammer eine weitere schlitzförmige Aussparung vorgesehen ist, die sich senkrecht zu der Frontwand in das Innere des Laborgeräts erstreckt, und in der der mindestens eine zweite Schwenkarm in der ersten Position (P1) des Türblattelements angeordnet ist.

Insbesondere ist in keiner Position des Türblattelements ein Schwenkarm der Schwenktüreinrichtung teilweise oder vollständig innerhalb der Kammer angeordnet.

Vorzugsweise weist das Türblattelement eine Heizeinrichtung auf, durch die das Türblattelement, insbesondere ein Sichtfenster des Türblattelements und/oder ein Wandteil der Türe, insbesondere ein der Kammeröffnung zugewandtes Wandteil, beheizbar ist.

Vorzugsweise weist das Türblattelement ein oder mehrere Versteifungsprofile auf.

Vorzugsweise ist um die Kammeröffnung herum eine elastomere Dichtung, insbesondere eine Silikondichtung, angeordnet, welche in der Position P1 des Türblattelements von einem thermischen, insbesondere polymeren, Isolationsmaterial des Türblattelements oder einem der Kammeröffnung zugewandten Wandteil des Türblattelements kontaktiert wird, das insbesondere ein Edelstahlblechteil ist und insbesondere beheizt ist.

Vorzugsweise ist das Laborgerät ein Inkubationsschüttler, insbesondere ein CO2-Inkubationsschüttler. Vorzugsweise weist der Laborschüttler eine Schubladeneinrichtung mit mindestens einem Schubladenelement auf, das beweglich in dem Vorrichtungsraum gelagert ist und mit dem die mindestens eine Antriebskomponente verbunden ist. Vorzugsweise ist das mindestens eine Schubladenelement zwischen einer ersten Position, in der das mindestens eine Schubladenelement im Vorrichtungsraum angeordnet ist, und einer zweiten Position bewegbar, in der das mindestens eine Schubladenelement zumindest teilweise aus dem Vorrichtungsraum ausgefahren ist

Eine optionale Anordnung von Antriebskomponenten auf einer Schublade führt zu einer optimalen Erreichbarkeit aller Antriebsteile zu Wartungszwecken. Die gute Erreichbarkeit des Antriebs kann auch zur Umstellung eines Misch-Orbits dienen. Somit ist es für den Anwender oder den technischen Service möglich, den Anwendungsbereich des Antriebes im Feld zu modifizieren - unterschiedliche Orbits dienen der optimalen Mischbarkeit verschiedener Probengefäße.

Die Plattformeinrichtung ist insbesondere aus dem Kammerinnenraum entnehmbar. Die Plattformeinrichtung ist insbesondere an mindestens einer Antriebskomponente montierbar und von dieser demontierbar, insbesondere an einer Übertragungseinrichtung, insbesondere einer Übertragungsplatte. Dazu ist mindestens ein Verbindungsmittel vorgesehen, welches die Plattformeinrichtung, insbesondere eine Subplattform der Plattformeinrichtung, mit der Antriebskomponente, insbesondere der Übertragungseinrichtung, insbesondere einer Übertragungsplatte, lösbar verbindet. Das mindestens eine Verbindungsmittel kann insbesondere zum werkzeuglosen Herstellen oder Lösen dieser Verbindung eingerichtet sein. Dazu kann das mindestens eine Verbindungsmittel eine Schraube mit Handdrehkopf beinhalten, eine Rasteinrichtung oder eine Schnellspanneinrichtung.

Die Plattformeinrichtung kann eine Subplattform beinhalten, die mittels dem mindestens einen Verbindungsmittel mit der Antriebskomponente, insbesondere der Übertragungsplatte, verbunden und/oder verbindbar ist.

Die Plattformeinrichtung kann eine Trageplattform beinhalten, welche dem Tragen der zu Schüttelnden Probengefäße in der Kammer dient.

Die Plattformeinrichtung ist jedenfalls im Betrieb des Laborschüttlers, also während der Schüttelbewegung, in der Kammer angeordnet.

Die Plattformeinrichtung kann eine Schieneneinrichtung beinhalten, mittels der die Trageplattform aus der Kammer herausfahrbar ist, wenn die Kammertüre geöffnet ist. Dadurch kann der Laborschüttler komfortable beladen und entladen werden. Zudem wird die Demontage der Plattformeinrichtung, insbesondere Subplattform, von der Übertragungseinrichtung vereinfacht. Die Schieneneinrichtung weist insbesondere erste Schienenelemente auf, die an der Subplattform gelagert sind, und zweite Schienenelemente, die an der Trageplattform gelagert sind. Die zweiten Schienenelemente können durch Gleitlagerung und/oder Wälzlagerung an den ersten Schienenelementen gelagert sein.

Der Vorrichtungsraum ist unterhalb der Plattformeinrichtung angeordnet. Dadurch ist der Vorrichtungsraum einfach separierbar vom Probenraum innerhalb der Kammer, in dem die Plattformeinrichtung und die Probengefäße auf der Plattformeinrichtung angeordnet sind. Der Vorrichtungsraum kann ein Raum innerhalb der Kammer sein. Vorzugsweise ist der Vorrichtungsraum ein Raum außerhalb der Kammer, insbesondere unterhalb der Kammer. "Unterhalb" bedeutet "in Richtung der Gravitation", da der Laborschüttler im bestimmungsgemäßen Betrieb so angeordnet ist, dass eine Trageplattform einen horizontalen Trägerbereich aufweist.

Der Vorrichtungsraum wird auch als Antriebsraum bezeichnet, da dort mindestens eine Antriebskomponente angeordnet ist. Es ist aber auch möglich, dass dort Komponenten angeordnet sind, die nicht zur Antriebsvorrichtung zu rechnen sind, beispielsweise elektronische Bauteile, zum Beispiel eine elektronische Steuereinrichtung des Laborschüttlers. Diese elektronischen Bauteile können mindestens eine Platine beinhalten.

Der Laborschüttler weist eine Schubladeneinrichtung mit mindestens einem Schubladenelement auf, das beweglich in dem Vorrichtungsraum gelagert ist und mit dem die mindestens eine Antriebskomponente verbunden ist und das insbesondere diese mindestens eine Antriebskomponente trägt. Das Schubladenelement ist im Betrieb des Laborschüttlers vorzugsweise durch Verbindungsmittel, insbesondere Schrauben, eine Rasteinrichtung oder eine Schnellspanneinrichtung, mit einer Gerätebasis des Laborschüttlers verbunden, insbesondere verbindbar. Vor dem Herausziehen des Schubladenelements aus dem Vorrichtungsraum ist diese durch das Verbindungsmittels erzeugte feste Verbindung zu lösen.

Das mindestens eine Schubladenelement ist zwischen einer ersten Position, in der das mindestens eine Schubladenelement im Vorrichtungsraum angeordnet ist, und einer zweiten Position bewegbar, in der das mindestens eine Schubladenelement aus dem Vorrichtungsraum ausgefahren ist. Vorzugsweise ist die Schubladeneinrichtung dazu eingerichtet, dass das Schubladenelement in der zweiten Position zu mindestens 50% aus dem Vorrichtungsraum herausziehbar ist, vorzugsweise zu mindestens 70%, vorzugsweise zu mindestens 80%, vorzugsweise zu mindestens 90%, oder vorzugsweise zu mindestens 95%, oder vorzugsweise vollständig. In den letzten beiden Fällen wird der Auszug als "Vollauszug" bezeichnet.

Vorzugsweise weist der Laborschüttler eine Gerätebasis auf, welche die restlichen Bestandteile des Laborschüttlers trägt. Die Gerätebasis ist insbesondere geeignet, mindestens einen weiteren Laborschüttler zu tragen, wenn der Laborschüttler stapelbar ausgebildet ist. Das Schubladenelement ist einerseits fest mit der Gerätebasis verbunden, andererseits insbesondere mittels einer Schieneneinrichtung beweglich an der Gerätebasis gelagert.

Vorzugsweise beinhaltet die Antriebsvorrichtung eine Antriebseinheit, insbesondere einen Motor, die mit der Gerätebasis fest verbunden ist. Die Antriebseinheit ist in diesem Fall nicht an dem Schubladenelement gelagert und wird somit auch nicht aus dem Laborschüttler herausbewegt, wenn das Schubladenelement von der ersten in die zweite Position bewegt wird.

Die Antriebsvorrichtung kann aber auch eine Antriebseinheit, insbesondere einen Motor, beinhalten, die mit dem mindestens einen Schubladenelement fest verbunden ist. Die Antriebseinheit ist in diesem Fall an dem Schubladenelement gelagert und wird somit auch aus dem Laborschüttler herausbewegt, wenn das Schubladenelement von der ersten in die zweite Position bewegt wird.

Vorzugsweise weist die Antriebsvorrichtung eine zur Bereitstellung einer Antriebsbewegung eingerichtete Antriebseinheit und eine zur Umwandlung der Antriebsbewegung in die Schüttelbewegung eingerichtete Getriebeeinrichtung auf, die mindestens ein Getriebeelement aufweist. Dabei sind die Antriebseinheit und das mindestens eine Getriebeelement Antriebskomponenten, von denen mindestens eine mit dem mindestens einen Schubladenelement verbunden sind.

Vorzugsweise wird die Kammer durch eine Kammerbodenwand begrenzt, welche den Innenraum der Kammer von dem vorzugsweise unterhalb der Kammerbodenwand vorgesehenen Vorrichtungsraum trennt und die zur Kopplung der Antriebsvorrichtung mit der Plattformeinrichtung eingerichtet ist, insbesondere, indem sie mindestens eine Bodenöffnung aufweist.

Vorzugsweise ist die Kammer durch eine Kammerbodenwand begrenzt und der Laborschüttler weist mindestens ein Verbindungselement, insbesondere eine Kopplungsstange auf, durch das die Plattformeinrichtung mit der mindestens einen Antriebskomponente lösbar verbunden ist. Vorzugsweise erstreckt sich das mindestens eine Verbindungselement durch mindestens eine Bodenöffnung der Kammerbodenwand, wenn die Plattformeinrichtung mit der mindestens einen Antriebskomponente verbunden ist.

Vorzugsweise verläuft die Schüttelbewegung parallel zu der Kammerbodenwand, also insbesondere horizontal, wobei mindestens eine Bodenöffnung der Kammerbodenwand vorzugsweise so dimensioniert ist, dass die der Schüttelbewegung entsprechende Relativbewegung von Verbindungselement und Kammerbodenwand ermöglicht wird.

Vorzugsweise ist die mindestens eine Bodenöffnung durch ein Dichtungselement abgedichtet, das zwischen Kammerbodenwand und Verbindungselement angeordnet ist. Vorzugsweise ist jede Bodenöffnung durch ein Dichtungselement abgedichtet, das zwischen Kammerbodenwand und Verbindungselement angeordnet ist.

Vorzugsweise ist das Dichtungselement mit dem Verbindungselement verbunden, vorzugsweise aber nicht mit der Kammerbodenwand verbunden ist oder vorzugsweise auch mit der Kammerbodenwand verbunden.

Vorzugsweise weist das Verbindungselement und/oder eine Antriebskomponente, insbesondere die Übertragungseinrichtung, mindestens ein Verbindungsmittel auf, insbesondere eine Schraube oder Langschraube, die insbesondere durch eine zentrale Bohrung im Verbindungselement geführt wird, oder eine Rast- oder Schnellspanneinrichtung. Durch das mindestens eine Verbindungsmittel ist das Verbindungselement vorzugsweise lösbar mit der Antriebskomponente, insbesondere der Übertragungseinrichtung, verbindbar, insbesondere während die Bodenöffnung durch das Dichtungselement abgedichtet ist.

Vorzugsweise weist das Verbindungsmittel ein Gewinde auf, das zur Herstellung einer Schraubverbindung zwischen dem Verbindungselement und der Übertragungseinrichtung eingerichtet ist und das insbesondere konzentrisch zu einer zentralen Längsachse des Verbindungselements angeordnet ist.

Vorzugsweise weist die mindestens eine Antriebskomponente, insbesondere ein Getriebeelement, eine Übertragungseinrichtung, insbesondere eine Übertragungsplatte auf, zum Tragen der Plattformeinrichtung. Mit der Übertragungseinrichtung ist insbesondere das mindestens eine Verbindungselement verbindbar.

Insbesondere ist die Übertragungseinrichtung mit dem mindestens einen Schubladenelement verbunden, wenn dieses zwischen der ersten und der zweiten Position bewegt wird.

Vorzugsweise beinhaltet die mindestens eine Antriebskomponente eine Riemenscheibe beinhaltet, die über einen Riemen mit der Antriebseinheit gekoppelt ist, die insbesondere neben der Schubladeneinrichtung angeordnet ist.

Vorzugsweise ist die Antriebseinheit ein Direktantrieb. Die Abtriebswelle des Direktantriebs ist vorzugsweis koaxial mit einer Antriebsscheibe, insbesondere Exzenterscheibe verbunden, um diese -insbesondere ohne Verwendung einer Getriebeeinrichtung- anzutreiben. Die Antriebseinheit ist in diesem Fall insbesondere auf dem Schubladenelement angeordnet. Die Antriebseinheit kann ein Elektromotor in Flachbauweise sein, dessen Höhe geringer ist als dessen Breite und/oder Tiefe. Insbesondere kann die Antriebseinheit ein scheibenförmiger Motor sein, insbesondere ein Scheibenläufermotor.

Die Antriebsscheibe weist vorzugsweise ein Übertragungselement auf, das exzentrisch zur Rotationsachse der Antriebsscheibe angeordnet ist und das insbesondere die Schüttelbewegung bewirkt. Das Übertragungselement verbindet vorzugsweise die Antriebsscheibe mit der Übertragungseinrichtung.

Vorzugsweise weist die mindestens eine Antriebskomponente einen oder mehrere bewegliche Sockelteile, auch bezeichnet als Lagerelemente oder Idler, auf, die insbesondere mit dem mindestens einen Schubladenelement und insbesondere mit der Übertragungseinrichtung bzw. der Übertragungsplatte verbunden sind, welche mittels der Übertragungsplatte die Plattformeinrichtung tragen.

Ein Idler (deutsch: Leerlaufrolle) ist insbesondere dazu eingerichtet, um die Plattformeinrichtung zu unterstützen, die durch die Exzenterscheibe angetrieben wird und eine Orbitalbewegung ausführt. Der Idler hat insbesondere keine aktive Antriebsfunktion, sondern dient vielmehr dazu, die Übertragungseinrichtung zu stabilisieren und zu unterstützen, während sie durch den Exzenter angetrieben wird.

Der Idler befindet sich typischerweise an einem Punkt entlang der Bahn der Übertragungseinrichtung und hilft dabei, die seitliche Bewegung zu stabilisieren und zu führen, während die Plattformeinrichtung die Schüttelbewegung durchführt. Sie trägt dazu bei, die Belastung auf das Übertragungselement und die Antriebskomponenten zu verringern und die Lebensdauer des Systems zu verlängern.

Vorzugsweise unterstützt der mindestens eine Idler die Übertragungseinrichtung und damit die Plattformeinrichtung, indem er eine stabile Führung entlang der Bahn der Orbitalbewegung gewährleistet und die exzentrische Bewegung des Exzenters stabilisiert.

Vorzugsweise weist Schubladeneinrichtung eine Schieneneinrichtung auf, mittels der das mindestens eine Schubladenelement mittels einer Gleit- oder Wälzlagerung beweglich im Vorrichtungsraum an der Gerätebasis gelagert ist. Die Schieneneinrichtung weist insbesondere erste Schienenelemente auf, die an der Gerätebasis gelagert sind, und zweite Schienenelemente, die an dem Schubladenelement gelagert sind. Die zweiten Schienenelemente können durch Gleitlagerung und/oder Wälzlagerung an den ersten Schienenelementen gelagert sein.

Vorzugsweise weist die Antriebsvorrichtung eine zur Bereitstellung einer Antriebsbewegung eingerichtete Antriebseinheit und eine zur Umwandlung der Antriebsbewegung in die Schüttelbewegung eingerichtete Getriebeeinrichtung auf, die mindestens ein Getriebeelement aufweist. Dabei sind die Antriebseinheit und das mindestens eine Getriebeelement Antriebskomponenten, von denen mindestens eine mit dem mindestens einen Schubladenelement verbunden sind. Die Antriebsbewegung ist insbesondere eine Rotationsbewegung der Abtriebswelle eines Elektromotors. Die Schüttelbewegung der Plattformeinrichtung ist derart, dass sich integral alle Punkte auf der Plattformeinrichtung in einer gemeinsamen, ebenen, orbitalen Bahn bewegen, definiert als Überlagerung zweier Translationen und in der Amplitude durch einen Exzenter.

Die Getriebeeinrichtung beinhaltet insbesondere solche beweglichen Komponenten, die in der kinematischen Kette zwischen der Antriebseinheit und den Verbindungselementen stehen, welche die Plattformeinrichtung mit der Antriebsvorrichtung verbinden.

Vorzugsweise wird die Kammer durch eine Kammerbodenwand begrenzt, welche den Innenraum der Kammer von dem vorzugsweise unterhalb der Kammerbodenwand vorgesehenen Vorrichtungsraum trennt und die zur Kopplung der Antriebsvorrichtung mit der Plattformeinrichtung eingerichtet ist, insbesondere, indem sie mindestens eine Bodenöffnung aufweist, insbesondere mehrere Bodenöffnungen, vorzugsweise zwei, drei oder vorzugsweise vier Bodenöffnungen.

Vorzugsweise ist die Kammer durch eine Kammerbodenwand begrenzt. Vorzugsweise weist der Laborschüttler mindestens ein Verbindungselement auf, insbesondere eine Kopplungsstange, durch das die Plattformeinrichtung mit der mindestens einen Antriebskomponente lösbar verbunden ist. Die Verbindung durch dieses Verbindungselement ist vorzugsweise form- und/oder kraftschlüssig. Vorzugsweise erstreckt sich das mindestens eine Verbindungselement durch mindestens eine Bodenöffnung der Kammerbodenwand, wenn die Plattformeinrichtung mit der mindestens einen Antriebskomponente verbunden ist. Vorzugsweise erstreckt sind mehrere Bodenöffnungen vorgesehen, durch die sich jeweils genau ein Verbindungsmittel erstreckt.

Vorzugsweise verläuft die Schüttelbewegung parallel zu der Kammerbodenwand, also insbesondere horizontal, wobei mindestens eine Bodenöffnung der Kammerbodenwand vorzugsweise so dimensioniert ist, dass die der Schüttelbewegung entsprechende Relativbewegung von Verbindungselement und Kammerbodenwand ermöglicht wird.

Vorzugsweise ist die mindestens eine Bodenöffnung durch ein Dichtungselement abgedichtet, das zwischen Kammerbodenwand und Verbindungselement angeordnet ist. Vorzugsweise ist jede Bodenöffnung durch ein Dichtungselement abgedichtet, das insbesondere zwischen Kammerbodenwand und Verbindungselement angeordnet ist.

Vorzugsweise ist das Dichtungselement mit dem Verbindungselement verbunden, vorzugsweise aber nicht mit der Kammerbodenwand verbunden ist oder vorzugsweise auch mit der Kammerbodenwand verbunden. Die Verbindung ist dabei jeweils vorzugsweise kraft/und oder formschlüssig und/oder stoffschlüssig.

Vorzugsweise weist das Verbindungselement und/oder eine Antriebskomponente, insbesondere die Übertragungseinrichtung, mindestens ein Verbindungsmittel auf, insbesondere eine Schraube oder Langschraube, die insbesondere durch eine zentrale Bohrung im Verbindungselement geführt wird, oder eine Rast- oder Schnellspanneinrichtung. Durch das mindestens eine Verbindungsmittel ist das Verbindungselement vorzugsweise lösbar mit der Antriebskomponente, insbesondere der Übertragungseinrichtung, verbindbar, insbesondere während die Bodenöffnung durch das Dichtungselement abgedichtet ist.

Vorzugsweise weist das Verbindungsmittel ein Gewinde auf, das zur Herstellung einer Schraubverbindung zwischen dem Verbindungselement und der Übertragungseinrichtung eingerichtet ist und das insbesondere konzentrisch zu einer zentralen Längsachse des Verbindungselements angeordnet ist.

Vorzugsweise weist die mindestens eine Antriebskomponente, insbesondere ein Getriebeelement, die Übertragungseinrichtung, insbesondere eine Übertragungsplatte, auf, zum Tragen der Plattformeinrichtung. Mit der Übertragungseinrichtung ist insbesondere das mindestens eine Verbindungselement verbindbar.

Insbesondere ist die Übertragungseinrichtung mit dem mindestens einen Schubladenelement verbunden, wenn dieses zwischen der ersten und der zweiten Position bewegt wird.

Vorzugsweise weist die Kammerbodenwand mindestens eine Bodenöffnung auf, die durch ein Dichtungselement verschlossen ist. Vorzugsweise weist der Laborschüttler mindestens ein Verbindungselement auf, das die mindestens eine Antriebskomponente mit der Plattformeinrichtung verbindet und das sich durch die mindestens eine Bodenöffnung sowie das Dichtungselement erstreckt.

Mittels der Dichtungselemente wird ein Kammerinnenraum ermöglicht, in dem keine Antriebskomponenten angeordnet sind und der leicht zu reinigen ist. Trotz der durch die Kammerbodenöffnungen entstehenden thermischen Schwachstellen verhindern die Dichtungselemente zu kühle Oberflächentemperaturen der Kammerbodenwand, die zu Kondensat führen würden. Die Dichtungselemente unterstützen zudem die geringe Bauhöhe des Laborschüttlers, da der Kammerinnenraum optimal auch in der Höhe mit großen Probengefäßen genutzt werden kann. Die Bauhöhe ist vor allem für Shaker, die gestapelt genutzt werden, von großem Nutzen, da auch die oberen Geräte noch ergonomisch bedienbar sein müssen. Zudem ist die optimale Höhenausnutzung wichtig bei Geräten, die den höheren Durchsatz bzw. die optimale Proteinausbeute bei der Expression in Suspensionszellen bedienen wollen.

Die beiden bevorzugten technischen Konzepte von Dichtungselementen mit Gleitlagerung verzichten auf ein zwischen Kammerbodenwand und Verbindungselement fest verbundenes Elastomerteil, um dem Verschleiß des Elastomers bei dessen, durch dauerhafte Schüttelbewegung bewirkter, hoher Belastung entgegenzusteuern.

Vorzugsweise weist das Dichtungselement ein elastomeres Material auf oder besteht daraus, insbesondere einem Silikonmaterial, vorzugsweise platinvernetztes Silikon, oder ein Fluorkautschuk.

Vorzugsweise ist das Dichtungselement ein flaches Bauteil, dessen maximale Ausdehnung in Richtungen parallel zur Kammerbodenwand größer ist als dessen maximale Ausdehnung gemessen senkrecht zur Kammerbodenwand.

Vorzugsweise weist das Dichtungselement einen, insbesondere zentralen, Durchgangskanal, mit insbesondere einer Durchtrittsöffnung auf, durch den/die das Verbindungselement durchtritt und an dem das Verbindungselement insbesondere dicht anliegt, und/oder wobei das Dichtungselement am Verbindungselement gelagert und/oder befestigt ist. Vorzugsweise handelt es sich um einen axialen Durchgangskanal, dessen Wanddicke größer ist als die Dicke einer sich radial erstreckenden Wand des Dichtungselements.

Vorzugsweise weist das Verbindungselement entlang seiner Längsachse A mindestens einen Befestigungsabschnitt mit einer insbesondere variierenden radialen Ausdehnung auf. Vorzugsweise kontaktiert das Dichtungselement, insbesondere dessen Durchtrittskanal, diesen Befestigungsabschnitt, und umschließt diesen insbesondere formschlüssig.

Vorzugsweise ist das Dichtungselement durch eine Gleitlagereinrichtung beweglich an der Kammerbodenwand gelagert, wobei
i) vorzugsweise die Gleitlagereinrichtung eine sich parallel zur Kammerbodenwand erstreckende Gleitfläche aufweist; ("axiales Gleitlager")
   oder alternativ,
ii) vorzugsweise die Gleitlagereinrichtung eine sich nicht parallel oder senkrecht zur Kammerbodenwand erstreckende Gleitfläche aufweist, ("radiales Gleitlager")

Vorzugsweise ist das Dichtungselement ein, insbesondere scheibenförmiges, Kappenelement ist, das die Kammerbodenöffnung, insbesondere während des laufenden Betriebs der Antriebsvorrichtung, überdeckt. Dies ist insbesondere nützlich bei der Gleitlagerung des Dichtungselements.

Vorzugsweise ist der längere Teil des Verbindungselements, gemessen entlang dessen Längsachse A, außerhalb der Kammer und unterhalb der Kammerbodenwand angeordnet. Dadurch wird ein kurzer Abstand zwischen Plattformeinrichtung und Kammerbodenwand ermöglicht, und die Nutzung des Kammerinnenraums wird optimiert.

Vorzugsweise ist das Dichtungselement an dem Verbindungselement befestigt und dazu eingerichtet, während der Schüttelbewegung an einer parallel zur Kammerbodenwand vorgesehenen Gleitfläche entlang zu gleiten, die an der Kammerwandöffnung, insbesondere kreisscheibenringfömig um diese herum, vorgesehen ist. Dieses Konzept wird als axiale Gleitlagerung bezeichnet. Vorzugsweise weist das Dichtungselement einen parallel zur ebenen Gleitfläche und um die Kammerwandöffnung verlaufenden Dichtungsringabschnitt auf, der beim Gleiten in Kontakt mit der Gleitfläche steht.

Vorzugsweise ist das Dichtungselement flach und weist insbesondere eine Gleitebene auf, die im Wesentlichen parallel zur Kammerbodenwand verläuft, wobei vorzugsweise das am Verbindungselement verankerte Dichtungselement dazu eingerichtet ist, Neigungsabweichungen zwischen der Gleitebene und der ebenen Kammerbodenwand durch eine Beweglichkeit des Dichtungselements auszugleichen, insbesondere indem das Dichtungselement mindestens einen elastisch verformbaren, insbesondere ringförmigen, Abschnitt aufweist oder vollständig elastisch verformbar ist.

Vorzugsweise weist das Verbindungselement und/oder die Antriebskomponente mindestens ein Verbindungsmittel auf, insbesondere eine Schraube, insbesondere Langschraube, die durch einen zentralen Hohlraum bzw. Durchgangskanal im Verbindungselement geführt wird. Vorzugsweise ist das Verbindungselement lösbar mit der Antriebskomponente verbindbar, insbesondere im Zustand, während die Bodenöffnung durch das Dichtungselement abgedichtet ist. Vorzugsweise beinhaltet das Verbindungsmittel ein Gewinde auf, das zur Herstellung einer Schraubverbindung zwischen dem Verbindungselement und der Antriebskomponente eingerichtet ist und das insbesondere konzentrisch zu einer zentralen Längsachse des Verbindungselements angeordnet ist.

Vorzugsweise ist an mindestens einer Antriebskomponente, insbesondere einem Getriebeelement, eine Übertragungseinrichtung, die ein- oder mehrteilig sein kann, insbesondere eine Übertragungsplatte, zum Tragen der Plattformeinrichtung angeordnet, mit der das mindestens eine Verbindungselement verbunden ist. Dieses Getriebeelement, insbesondere die Übertragungseinrichtung, ist insbesondere außerhalb der Kammer angeordnet.

Vorzugsweise ist unterhalb der Kammer, benachbart oder angrenzend an die Kammerbodenwand bzw. die vorzugsweise dort angeordneten Heizwendel, eine Isoliermaterialschicht aus einem thermisch isolierenden Material angeordnet ist, das insbesondere eine Isoliermaterialöffnung aufweist, durch die das Verbindungselement durchtritt. Die Isoliermaterialöffnung liegt insbesondere konzentrisch zur Kammerbodenöffnung. Vorzugsweise ist ein mit dem Verbindungselement verbundenes Isolierelement vorgesehen ist, das gemeinsam mit dem Verbindungselement gegenüber der Isoliermaterialschicht beweglich ist und das die Isoliermaterialöffnung in axialer Richtung abdeckt oder verschließt, insbesondere auch während der Schüttelbewegung.

Vorzugsweise weist der Laborschüttler eine Heizeinrichtung, die insbesondere Heizwendel - vorzugsweise an der Kammeraußenseite- beinhaltet, und eine elektrische Steuereinrichtung auf, die dazu programmiert ist, ein Hochtemperaturprogramm auszuführen, durch das die Steuereinrichtung dazu programmiert ist, dass der durch das mindestens eine Dichtungselement abgedichtete Kammerinnenraum mittels der Heizeinrichtung für einen vorbestimmten Zeitraum auf eine vorbestimmte Temperatur aufgeheizt wird, wobei der Zeitraum zwischen 1 Minute und 12 Stunden, insbesondere 20 min bis 5 Std., betragen kann, und wobei die Temperatur zwischen 90 °C und 140°C betragen kann.

Die Erfindung betrifft auch ein Laborgerät zur Inkubation von in Probengefäßen (130) enthaltenen flüssigen Laborproben, insbesondere Inkubationsschüttler, aufweisend
- eine Kammer (2), die mindestens eine Kammerwand aufweist und eine Kammeröffnung (2a) zum Einstellen und Herausnehmen der Probengefäße (130) in einen Innenraum (3) der Kammer,
- eine Heizeinrichtung (190), die zum Beheizen der Kammer vorgesehen ist, die mindestens einen Heizwendel aufweist, der an der Außenseite der mindestens einen Kammerwand angeordnet ist,
   dadurch gekennzeichnet, dass
- die mindestens eine Kammerwand mindestens einen ersten Flächenbereich aufweist, in dem die von dem mindestens ein Heizwendel abgegebene Heizleistung größer ist als in einem zweiten Flächenbereich, insbesondere indem der mindestens ein Heizwendel im ersten Bereich mit einer höheren Flächendichte verlegt ist.

Die Heizleistung wird in Watt angegeben. Sie kann bei einem Heizdrahtabschnitt über eine elektrische Messung gemessen werden. Der bei diesen Angaben zugrunde gelegte Flächenbereich beträgt in der Praxis insbesondere zwischen 10 und 50 Quadratdezimeter. Bei der Inkubationskammer liegen die senkrechten Abstände von parallel verlegten Drähten in den zentralen Flächenbereichen (zweite Flächenbereiche) der Boden-, Seiten, Rück- und Deckenwand zwischen 3 und 15 cm, insbesondere zwischen 4 und 10 cm. In den Randbereichen und nahe von Öffnungen der Kammer (erste Flächenbereiche) sind die Abstände vorzugsweise geringer als in den zentralen Flächenbereichen.

Vorzugsweise ist die Wärmestromdichte, angegeben in Watt/Quadratmeter, in dem ersten Flächenbereich größer als in dem zweiten Flächenbereich.

Der Heizdraht bzw. Heizwendel ist insbesondere auf die Kammerwand geklebt, insbesondere mittels eines Klebebands, insbesondere metallischen Klebebands, insbesondere Aluminiumklebeband.

Vorzugsweise ist der Flächenbelegungsanteil durch den mindesten einen Heizwendel, also die vom Heizwendel auf der Oberfläche belegte Fläche A_H geteilt durch die Flächeneinheit A, also A_H / A, in dem ersten Flächenbereich größer als in dem zweiten Flächenbereich. Die Größe A wird auch als Bezugsflächengröße bezeichnet und im Zusammenhang mit den Figuren weiter erläutert.

Vorzugsweise ist die pro Flächeneinheit auf der Oberfläche verlegte Länge des mindestens einen Heizwendel, gemessen in Meter pro Quadratmeter, in dem ersten Flächenbereich größer als in dem zweiten Flächenbereich.

Bevorzugt ist jeweils, den Anteil der Länge von Heizdraht pro planarer Fläche (zum Beispiel zentral im Deckenwandbereich der Kammer) -als zweitem Flächenbereich - mit dem Anteil der Länge von Heizdraht im Kammerwandrandbereich und/oder einer Kammerwandöffnung und/oder einem Kammerwandkrümmungsbereich -als erstem Flächenbereich- vergleichen.

Vorzugsweise liegt der erste Flächenbereich näher an einem Rand der Kammerwand, einer Öffnung der Kammerwand und/oder einem Krümmungsbereiche, insbesondere einer Ecke, der Kammer, als der zweite Flächenbereich. In den genannten Bereichen wird im Vergleich zur planaren Fläche der Kammerwand mehr Wärme an die Umgebung abgeführt, was durch die höhere Heizwendeldichte bzw. höhere Heizleistung kompensierbar ist. Im Resultat wird eine homogenere Kammertemperatur erreicht und das Risiko der Kondensatbildung an ersten Flächenbereichen vermieden.

Vorzugsweise weist das Laborgerät eine elektrische Steuereinrichtung auf, insbesondere eine Datenverarbeitungseinrichtung, und ist vorzugsweise dazu programmiert, eine Temperatur einer Kammer, insbesondere der Kammerwand, zu erfassen und insbesondere in Abhängigkeit von dieser Temperatur die Leistung der Heizeinrichtung einzustellen. Vorzugsweise ist die Steuereinrichtung dazu programmiert, eine Temperatur der Heizeinrichtung, auf eine gewünschte, insbesondere konstante, Zieltemperatur zu regeln. Vorzugsweise ist die Steuereinrichtung dazu programmiert, einen Heizregelkreis zu bilden, der dazu eingerichtet ist, die mittels eines Temperatursensors gemessene Temperatur eines Heizelements des Verdampfers auf eine konstante Zieltemperatur zu regeln, bei der ein mit dem Heizelement in Kontakt kommendes Wasservolumen verdampft wird und dabei dem Heizelement Wärme entzieht,

Die elektronische Steuereinrichtung ist vorzugsweise dazu programmiert, mindestens eine Funktion der Beleuchtungseinrichtung zur Beleuchtung des Kammerinnenraums zu steuern, insbesondere die Dauer und/oder Intensität und/oder Farbe und/oder in Abhängigkeit von Sensorsignalen, insbesondere dem Signal eines Türsensors des Inkubators.

Die Funktionen der Steuereinrichtung sind insbesondere durch Programmcode und/oder durch elektronische Schaltkreise implementiert. Die Steuereinrichtung kann einen Mikrocontroller, eine Recheneinheit (CPU) zum Verarbeiten von Daten bzw. einen Mikroprozessor aufweisen, die jeweils der Datenverarbeitungseinrichtung zugeordnet sein können.

Die Steuereinrichtung kann aber auch durch eine solche Steuereinrichtung gebildet sein, die mindestens eine, mehrere oder alle Funktionen des Laborgeräts steuert. Eine der Funktionen des Laborgeräts ist insbesondere die Regelung der Temperatur in der Inkubationskammer des Laborgeräts, oder die Regelung der Gaszusammensetzung in der Inkubationskammer, insbesondere der CO2-Konzentration. Eine der Funktionen des Laborgeräts ist insbesondere auch die Steuerung eines Benutzerschnittstellenmoduls des Laborgeräts, das dem Benutzer Informationen anzeigt, insbesondere über Sensorwerte zu in/an der Inkubationskammer gemessenen physikalischen oder chemischen Größen.

Die Erfindung betrifft auch ein Verfahren zur Behandlung des Kammerinnenraums eines Laborschüttlers gemäß einem der vorangehenden Ansprüche, der eine Heizeinrichtung und eine elektrische Steuereinrichtung aufweist, die dazu programmiert ist, ein Hochtemperaturprogramm auszuführen, durch das die Steuereinrichtung dazu programmiert ist, dass der durch das mindestens eine Dichtungselement abgedichtete Kammerinnenraum mittels der Heizeinrichtung für einen vorbestimmten Zeitraum auf eine vorbestimmte Temperatur aufgeheizt wird, wobei der Zeitraum zwischen 1 Minute und 12 Stunden, insbesondere 20 min bis 5 Std., betragen kann, und wobei die Temperatur zwischen 90 °C und 140°C, insbesondere bis zu 180 °C oder bis 200 °C, betragen kann, wobei das Verfahren den Schritt aufweist:
- Aufheizen des Kammerinnenraums mittels der Heizeinrichtung für einen vorbestimmten Zeitraum auf eine vorbestimmte Temperatur, wobei der Zeitraum zwischen 1 Minute und 12 Stunden, insbesondere 20 min bis 5 Std., betragen kann, und wobei die Temperatur zwischen 90 °C und 140°C, insbesondere bis zu 180 °C oder bis 200 °C, betragen kann.

Vorzugsweise weist das Dichtungselement mindestens eine Exzenterscheibe auf und insbesondere eine Gleitfläche mit radialer Ausrichtung.

Vorzugsweise weist das Dichtungselement mindestens eine Exzenterscheibe auf, die mindestens eine Gleitfläche mit radialer Ausrichtung, insbesondere mit Ausrichtung radial nach außen, aufweist.

Vorzugsweise weist das Dichtungselement mindestens eine erste Exzenterscheibe auf, die mindestens eine Gleitfläche mit radialer Ausrichtung, insbesondere mit Ausrichtung radial nach außen, aufweist, und vorzugsweise weist das Dichtungselement mindestens eine zweite Exzenterscheibe auf, die mindestens eine Gleitfläche mit radialer Ausrichtung, insbesondere mit Ausrichtung radial nach außen, aufweist. Diese Anordnung wird auch als Zweifach-Exzenterscheibe bezeichnet. Das Verbindungselement ist dabei vorzugsweise um seine Längsachse drehbar azentrisch in der zweiten Exzenterscheibe gelagert, vorzugsweise mittels eines Gelenklagers.

Vorzugsweise weist das Dichtungselement einen Dichtungsringabschnitt auf, der parallel zur Gleitfläche verläuft, die gegenüber der Kammerbodenwand senkrecht verläuft oder zumindest geneigt ist und konzentrisch zur Kammerwandöffnung verläuft, und der beim Gleiten in Kontakt mit der Gleitfläche steht.

Vorzugsweise ist das Dichtungselement flach und weist eine Hauptebene auf, die im Wesentlichen parallel zur Kammerbodenwand verläuft, wobei das am Verbindungselement verankerte Dichtungselement mindestens einen elastisch verformbaren, insbesondere ringförmigen Abschnitt aufweist oder vollständig elastisch verformbar ist.

Vorzugsweise weist das Dichtungselement magnetische Abschnitte auf, durch deren magnetische Anziehungskraft das Dichtungselement in Richtung der Kammerbodenwand gezogen wird.

Vorzugsweise ist eine radiale Gleitfläche an einem kreisringförmigen Einsatzelement, insbesondere einer Lagerhülse, vorgesehen, das an der Kammerwandöffnung befestigt ist und insbesondere in diese hineinragt.

Vorzugsweise weist das Dichtungselement einen gekrümmten Wandabschnitt auf, der insbesondere als ringförmige Wanne gestaltet ist, in deren Zentrum ein Durchgangskanal, bzw. eine Durchtrittsöffnung für den Durchtritt des Verbindungselementes vorgesehen ist.

Vorzugsweise ist ein Halteringelement vorgesehen, mit dem das Dichtungselement an der Kammerbodenöffnung befestigt ist, und der sich insbesondere mit einem hohlzylinderförmigen Abschnitt in Richtung der Vorrichtungsraums erstreckt.

Der Laborschüttler zum Schütteln von Laborproben ist insbesondere zum Temperieren der Laborproben eingerichtet. Solche Geräte werden elektrisch betrieben und weisen einen Spannungsanschluss auf. Der Laborschüttler temperiert die Laborproben, das heißt, er hält das Gehäuseinnere und damit die dort lagernden Laborproben im Rahmen von Toleranzen durch eine Temperaturregelung auf einer insbesondere vom Benutzer einstellbaren Solltemperatur. Diese kann über der Raumtemperatur (Umgebungstemperatur) liegen, wie dies bei einem Wärmeschrank oder Inkubator der Fall ist, oder kann unter der Raumtemperatur liegen, wie dies bei einem Kühlschrank oder Gefrierschrank der Fall ist. Bei einem als Klimalaborschüttlerausgebildeten Laborschüttler wird vorzugsweise auch ein im Inneren des Gehäuses vorherrschender Klimaparameter im Rahmen von Toleranzen geregelt. Dieser Klimaparameter kann die Luftfeuchtigkeit sein, und/oder eine Gaskonzentration, z.B. eine CO2, O2 und/oder N2-Konzentration. Ein solcher Klimalaborschüttler ist beispielsweise ein Laborschüttler zum Schütteln von Laborproben, insbesondere mit lebenden Zellkulturen, mit Inkubatorfunktion, auch bezeichnet als Inkubationsschüttler.

Typische Merkmale solcher Laborschüttler können eine oder mehrere der folgenden Merkmale sein:
Temperaturkontrollierbarkeit der Kammer: Beheizung auf maximal 60 oder 80 °C für die Zellkultur.
Drehzahlbereich der Schüttelbewegung: (25 - 500, -1000 rpm).
Gehäuseformat derart, dass eine Aufstellbarkeit im Labor (auf dem Labortisch, unter dem Labortisch, stapelbare Standmodelle) möglich ist.
Stapelbarkeit des Gehäuses (2 oder 3 oder mehr übereinander).
Kapazität und Durchsatz: Gefäßtyp, Größe und Kapazität.
Beladungsart (von vorne oder von oben).
CO2-Regelung.
Photosynthetisches Licht.

Besonders bevorzugt ist der Laborschüttler dazu eingerichtet, mithilfe einer Temperiereinrichtung und/oder einer Heizeinrichtung ein Hochtemperaturverfahren im Inneren der Kammer zum Sterilisieren des Kammerinnenraums durchzuführen, bei dem die Kammer für einen Zeitraum von mehreren Sekunden (z.B. von 1, 2, 5, 10, 30 Sekunden) bis mehreren Minuten (z.B. bis zu 1, 2, 3 5, 10, 30, 60, 120, 240, 480 oder 600 Minuten) einer Temperatur von zwischen 150°C und 200°C, vorzugsweise mindestens 180° C ausgesetzt ist, ohne dass der Auszugsmechanismus, vorzugsweise inklusive der aufgesetzten Probenplattform, entnommen werden muss. Besonders bevorzugt ist der Laborschüttler, insbesondere eine elektronische Steuerungseinrichtung, welche insbesondere die Temperiereinrichtung und/oder die Heizeinrichtung steuert, dazu eingerichtet, die Kammer für einen Zeitraum von mehr als einer Stunde, insbesondere für einen Zeitraum von mehreren Stunden, z.B. für einen Zeitraum innerhalb eines Zeitintervalls von 2, 3, 4, 5, 6 ,7, 8, 9 oder 10 Stunden, einer Zieltemperatur von zwischen 150°C und 200°C, vorzugsweise mindestens 180° C auszusetzen. Dabei benötigt die Kammer in der Regel eine Aufwärmzeit, um die Zieltemperatur zu erreichen und eine Abkühlzeit, um von der Zieltemperatur wieder auf eine Normalbetriebstemperatur abzukühlen. Insbesondere ist der Auszugsmechanismus bei der Durchführung des Hochtemperaturzyklus in der verschlossenen Kammer angeordnet. Der Auszugsmechanismus und die Probenplattform sind insbesondere aus entsprechend hochtemperaturbeständigem Material gefertigt, insbesondere einem Edelstahl, oder Aluminium, insbesondere anodisiertem Aluminium.

Die Laborschüttler weist vorzugsweise ein Gehäuse auf. Das Gehäuse ist vorzugsweise ein äußeres Gehäuse, dessen Gehäusewände mit der Umgebung in Kontakt stehen. Die Gehäusetüre kann entsprechend eine äußere Gehäusetüre sein, die in der Verschlussposition an die Umgebung grenzt.

Die Gehäusetüre weist insbesondere eine Scharniereinrichtung auf, welche die Gehäusetüre schwenkbar mit dem Gehäuse verbindet. Eine solche Schwenktüre wird durch eine Rotation zwischen einer geöffneten Position und der Verschlussposition bewegt. Die Scharniereinrichtung kann insbesondere an der -im bestimmungsgemäßen Gebrauch des Laborschüttlers - vertikal orientierten Außenkante eines quaderförmigen Gehäuses liegen, welche an die Gehäuseöffnung angrenzt. Die Bodenplatte eines quaderförmigen Gehäuses ist im bestimmungsgemäßen Gebrauch des Laborschüttler horizontal angeordnet, die Seitenwände des Gehäuses sind insbesondere vertikal angeordnet, und die Deckplatte des Gehäuses ist insbesondere der Bodenplatte gegenüberliegend horizontal angeordnet.

Eine Datenverarbeitungseinrichtung ist vorzugsweise Bestandteil der elektrischen Steuereinrichtung, die Funktionen des Laborschüttlers steuert und die der Laborschüttler vorzugsweise aufweist. Die Funktionen der Steuereinrichtung sind insbesondere durch elektronische Schaltkreise implementiert. Die Steuereinrichtung kann einen Mikrocontroller, eine Recheneinheit (CPU) zum Verarbeiten von Daten und/oder einen Mikroprozessor aufweisen, die jeweils die Datenverarbeitungseinrichtung beinhalten können. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird. Ein solches Steuerungsverfahren kann den zeitlichen Verlauf einer Schüttelbewegung definieren, die mittels der Schütteleinrichtung durchgeführt werden kann. Diese Schüttelbewegung ist insbesondere definiert durch Richtung(en) von Translationsbewegungen und/oder Amplitude(n) von aufeinanderfolgenden Bewegungsabschnitten, die in einer x-y-Ebene durchgeführt werden. Diese x-y-Ebene ist in der Regel parallel zur Probenplattform und/oder eines Kammerbodens. Bevorzugte Durchmesser einer in einer x-y-Ebene ausgeführten Schüttelbewegung liegen zwischen 0 und 5,08 cm (2 Inch) oder bis 7.62 cm (3 Inch). Die Schütteleinrichtung, insbesondere ein Orbitalantrieb, ist vorzugsweise für eine Schüttelbewegung mit einem maximalen Durchmesser zwischen 0 und 5,08 cm (2 Inch) oder bis 7.62 cm (3 Inch) eingerichtet. Die Funktionen des Laborschüttlers und/oder der Steuereinrichtung können in Verfahrensschritten beschrieben werden. Sie können als Bestandteile des Steuerungsprogramms realisiert sein, insbesondere als Unterprogramme des Steuerungsprogramms.

Vorzugsweise ist der Laborschüttler ein Inkubationsschüttler. Der Inkubationsschüttler ist dann auch als Labor-Inkubator betreibbar und ist damit ein Gerät, mit dem kontrollierte Klimabedingungen für verschiedene biologische Entwicklungs- und Wachstumsprozesse geschaffen und erhalten werden können. Er dient insbesondere der Schaffung und Erhaltung eines Mikroklimas mit geregelten Gas-, und/oder Luftfeuchtigkeits- und/oder Temperatur-Bedingungen in der Kammer, wobei diese Behandlung zeitabhängig sein kann. Der Inkubationsschüttler kann insbesondere einen Zeitgeber aufweisen, insbesondere eine Zeitschaltuhr, eine als Heiz- und/oder Kühleinrichtung ausgeführte Temperiereinrichtung und vorzugsweise eine Einstellung für die Regelung eines der Kammer zugeführten Austauschgases, eine Einstelleinrichtung für die Zusammensetzung des Gases in der Kammer des Inkubationsschüttler, insbesondere zur Einstellung des CO₂ und/oder des O₂ und/oder des N₂-Gehalts Gehalts des Gases und/oder eine Einstelleinrichtung zur Einstellung der Luftfeuchtigkeit in der Kammer des Inkubationsschüttler.

Der Inkubationsschüttler weist insbesondere die Inkubatorkammer (=Kammer) auf, ferner vorzugsweise eine Regeleinrichtung mit mindestens einem Regelkreis, dem als Stellglied die mindestens eine Temperiereinrichtung und als Messglied mindestens ein Temperatursensor zugeordnet sind. Je nach Ausführungsform kann darüber auch die Luftfeuchte geregelt werden, wobei vorzugsweise die Luftfeuchtigkeit durch einen Luftfeuchte-Sensor (rH-Sensor) in der Kammer gemessen wird und die Luftfeuchtigkeit insbesondere Eingangsgröße des Regelkreises ist. Zur Luftbefeuchtung der Kammer kann darin eine mit Wasser gefüllte Wanne in der Inkubatorkammer vorgesehen sein, die geheizt oder gekühlt werden kann, um über die Verdunstung die Luftfeuchtigkeit einzustellen. Bevorzugter ist aber, eine Verdampfervorrichtung im Außenbereich der Kammer vorzusehe, die bedarfsweise Dampf erzeugt und durch eine Dampfeintrittsöffnung der Kammerwand der Kammer zuführt. Diese Dampfzuführung ist vorzugsweise mittels der Steuereinrichtung geregelt. CO₂- Inkubationsschüttler dienen insbesondere der Kultivierung tierischer bzw. humaner Zellen.

Die Steuerungseinrichtung kann dazu eingerichtet sein, dass ein Programmparameter oder ein Steuerungsparameter des Laborschüttlers, insbesondere des Inkubationsschüttlers, automatisch in Abhängigkeit von anderen Daten gewählt wird. Eine von einem Steuerungsparameter gesteuerte Behandlung der mindestens einen Zellkultur in mindestens einem Zellkulturbehälter entspricht bei einem Inkubator insbesondere einer Klimabehandlung, der die mindestens eine Zellkultur unterzogen wird. Mögliche Parameter, insbesondere Programmparameter, insbesondere Nutzerparameter, die zur Beeinflussung einer Klimabehandlung verwendet werden, definieren insbesondere die Temperatur der Kammer, in der die mindestens eine Probe inkubiert wird, die relative Gaskonzentration von O₂- und/oder CO₂ und/oder N₂ in der Kammer, die Luftfeuchtigkeit im in der Kammer und/oder mindestens einen Ablaufparameter, der den Ablauf, insbesondere die Reihenfolge, eines aus mehreren Schritten bestehenden Inkubationsbehandlungsprogramms und/oder Schüttelprogramms beeinflusst oder definiert.

Die Temperiereinrichtung kann eine kombinierte Heiz- / Kühleinrichtung sein. Sie ist vorzugsweise nur eine Heizeinrichtung. Diese kann insbesondere die Wärme über einen elektrischen Widerstandsdraht erzeugen. Vorzugsweise ist der Widerstandsdraht an der Außenseite mindestens einer, mehrerer oder aller Kammerwände angebracht, welche die Kammer bilden.

Die Laborschüttler bzw. der Inkubationsschüttler kann genau eine Kammer aufweisen, kann aber auch mehrere Kammern aufweisen, deren Atmosphäre (Temperatur, relative Gaskonzentration, Luftfeuchte) insbesondere individuell oder gesammelt einstellbar sein kann. Eine typische Größe des Inneren einer Kammer liegt zwischen 50 und 400 Litern, wobei auch für besondere Anwendungen (IVF) kleinere Kammergrößen möglich sind, insbesondere 10 bis 49 Liter.

Weitere bevorzugte Ausgestaltungen eines erfindungsgemäßen Laborschüttlers lassen sich der Beschreibung der Ausführungsbeispiele gemäß den Figuren entnehmen. Dabei bezeichnen gleiche Bezugszeichen auch im Wesentlichen gleiche Bauteile:

Es zeigen:
Fig. 1a zeigt eine perspektivische seitlich-frontale Ansicht eines erfindungsgemäßen Laborschüttlers gemäß Ausführungsbeispiel.
Fig. 1b zeigt den Laborschüttler der Fig. 1a, mit einer abgenommenen Seitenwand und den seitlich der Kammer in einer Elektronikkammer angeordneten Komponenten.
Fig. 1c zeigt den Laborschüttler der Fig. 1a, mit geöffneter Schwenktüre, wobei die Dichtungselemente, Verbindungselemente, Subplattform und Plattform sowie Probengefäße aus der Kammer entfernt sind und deshalb nicht dargestellt sind.
Fig. 1d zeigt den Laborschüttler der Fig. 1c, mit abgenommener Frontplatte, hinter der sich der, unterhalb der Kammer gelegene, Antriebsraum befindet.
Fig. 2a bis Fig. 2f zeigen, jeweils in perspektivischer Schrägansicht, verschiedene Positionen der Schwenktüreinrichtung bzw. der Schwenktüre des Laborschüttlers der Figur 1a, zwischen den, und einschließlich der, Positionen P1 und P2, bei nicht gezeigter Gehäuseseitenwand.
Fig. 3a bis 3c zeigen, in einer Seitenansicht, verschiedene Positionen der Schwenktüre und der Schwenkarme des Laborschüttlers der Figur 1a, bei nicht gezeigter Gehäuseseitenwand.
Fig. 4a zeigt, in einer Seitenansicht, einen Stapel mit drei Laborschüttlern gemäß der Figur 1a.
Fig. 4b zeigt in perspektivischer Ansicht ein seitliches Rahmenteil als Bestandteil der Trägereinrichtung des Laborgeräts der Fig. 1a.
Fig. 5a und 5b zeigen jeweils, in zwei verschiedenen Schrägansichten und von hinten, die Schwenktüre mit Schwenkarmen und Gasfedern des Laborschüttlers der Figur 1a, bei nicht gezeigter Gehäuseseitenwand.
Fig. 5c zeigt ein Detail einer Aufsicht auf die Schwenktüre mit Kopplungsstange des Laborschüttlers der Figur 1a.
Fig. 5d zeigt ein Detail einer Schnittansicht senkrecht durch das Türblattelement der Fig. 5a, 5b.
Fig. 6a und 6b zeigen eine Schnittansicht durch den Laborschüttler der Figur 1a entlang der in Fig. 1a gezeigten Linie A-A, bei nicht gezeigter Gehäuseseitenwand.

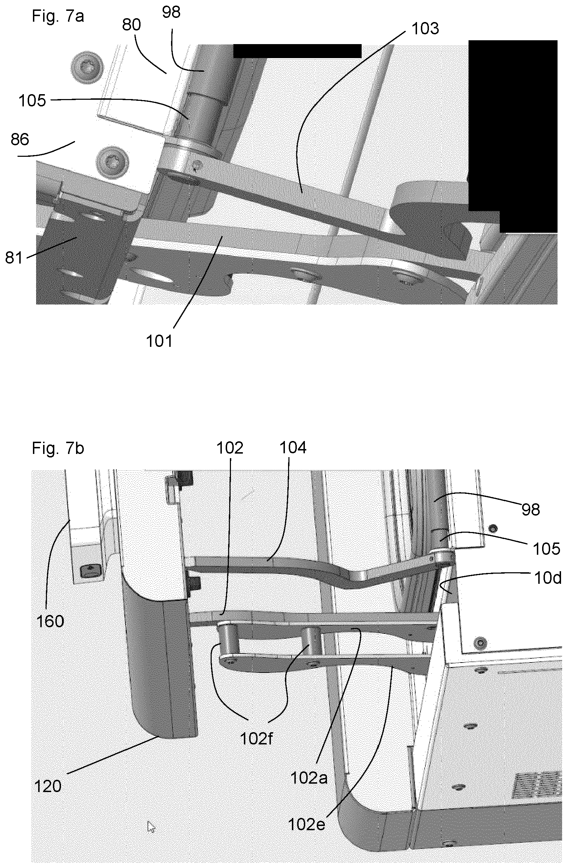

Fig. 7a zeigt eine perspektivische Schrägaufsicht auf erste Schwenkarme der Schwenktüre des Laborschüttlers der Figur 1a, an einer ersten Seite der Schwenktüre.
Fig. 7b zeigt eine perspektivische Schrägaufsicht auf zweite Schwenkarme der Schwenktüre des Laborschüttlers der Figur 1a, an einer zweiten Seite der Schwenktüre, welche der ersten Seite gegenüberliegt.
Fig. 8a zeigt, in einer Seitenansicht, eine Position der Schwenktüre und der Schwenkarme des Laborschüttlers der Figur 1a, wobei der Verlauf des unteren ersten Schwenkarms sichtbar gemacht ist.
Fig. 8b zeigt den unteren ersten Schwenkarm der Fig. 8a.
Fig. 8c zeigt den ersten oberen Schwenkarm und den zweiten oberen Schwenkarm des Laborschüttlers der Figur 1a, wobei die Schwenkarme durch eine Kopplungsstange starr verbunden sind.
Fig. 9a zeigt perspektivisch eine Rückansicht des Laborschüttlers der Figur 1a, mit abgenommenen Rückwänden und herausgenommener Isolationsschicht.
Fig. 9b zeigt die Rückansicht der Fig. 9a, mit eingesetzter Isolationsschicht und rückseitigem Spritzwasserschutz.
Figur 9c zeigt einen vertikalen Schnitt durch den Laborschüttler der Figur 1a, senkrecht zur Schwenktüre.

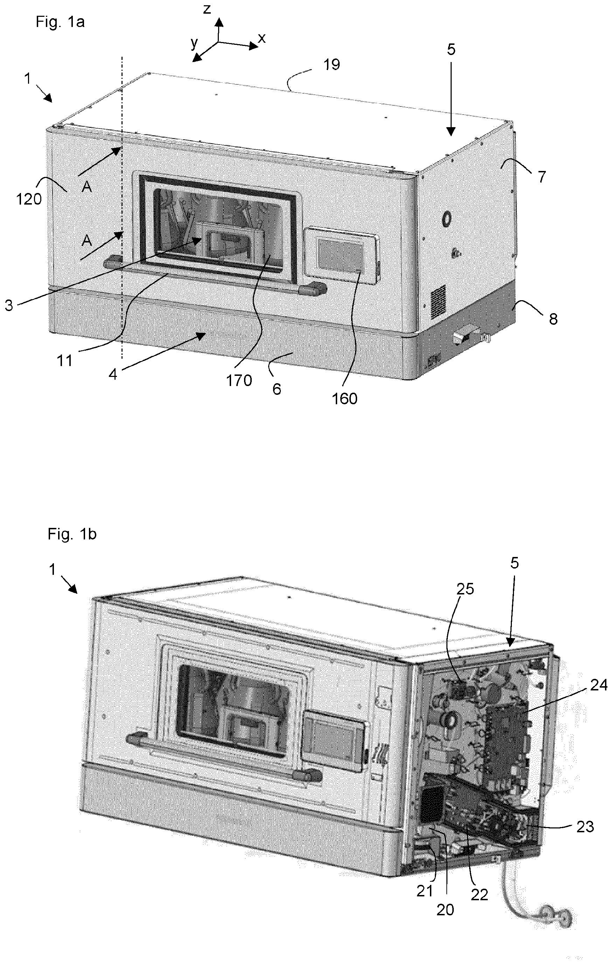

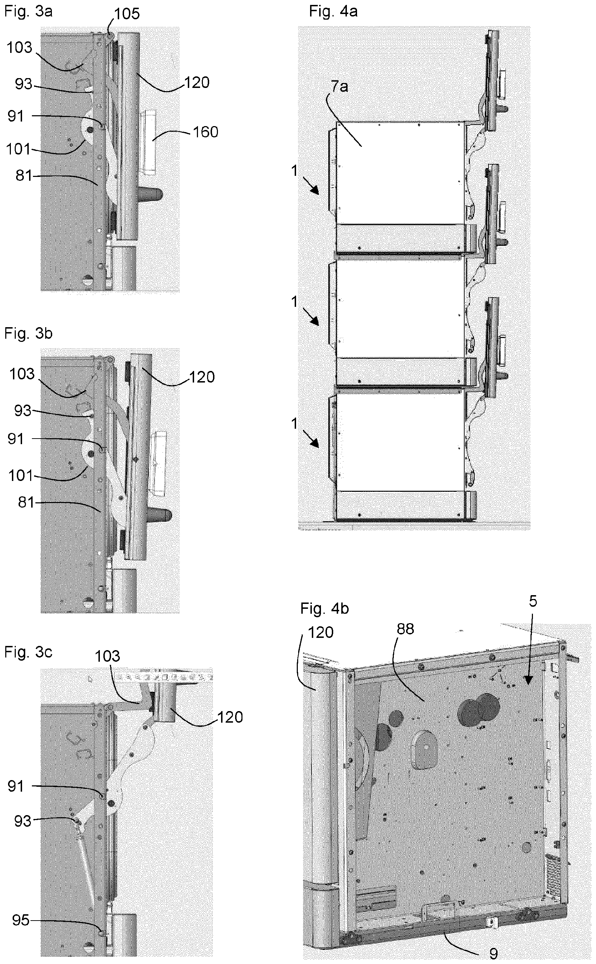

Fig. 1a zeigt eine perspektivische seitlich-frontale Ansicht eines erfindungsgemäßen Laborgeräts 1, hier eines Laborschüttlers 1. Es handelt sich um einen insbesondere stapelbaren (siehe Figur 4), CO2-Inkubationsschüttler 1. Der Kammerinnenraum 3 des Laborschüttlers fasst 220 I (Angabe des Nutzraums), bei einer insgesamt geringen Bauhöhe und geringen Breite des Laborschüttlers. Der Kammerinnenraum 3 enthält keine Bestandteile der Schwenktüreinrichtung 100 und steht deshalb in voller Breite für die Nutzbeladung zur Verfügung. Die Schwenktüreinrichtung 100 kann für die Anordnung außerhalb des Kammerinnenraums optimiert werden. Insbesondere müssen Bestandteile der Schwenktüreinrichtung, insbesondere die Schwenkarme, nicht separat gekapselt werden, wie dies zu Zwecken der Reinigbarkeit bei im Stand der Technik bekannten Geräten der Fall ist. Dadurch kann die Schwenktüreinrichtung besonders raumsparend gestaltet werden, insbesondere indem die Schwenkarme plattenförmige Bauteile sind. Dies kommt einer insgesamt relativ geringen Gerätebreite des Laborgeräts zugute.

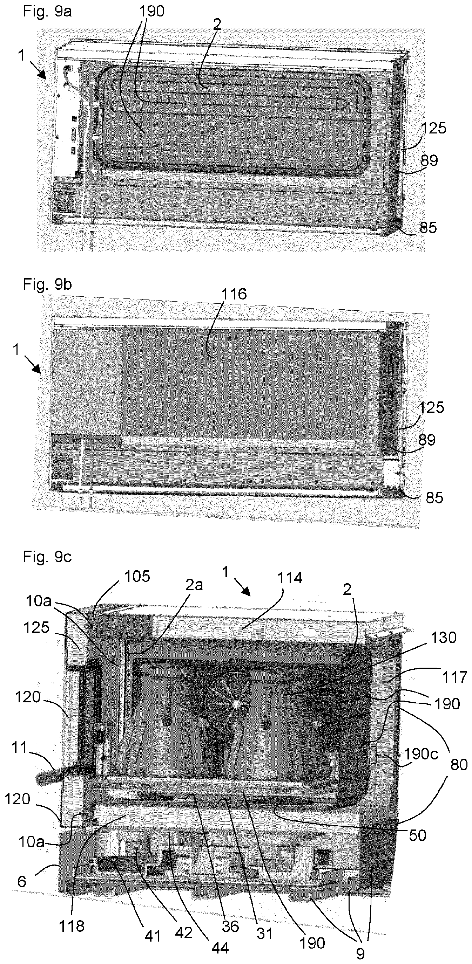

Eine geringe Bauhöhe wird insbesondere dadurch ermöglicht, dass die Dichtungselemente 50 zwischen Antriebsraum 4 (auch: Vorrichtungsraum 4) und Kammerinnenraum 3 in Flachbauweise ausgeführt sind und auch der Antriebsraum flach gehalten ist. Gezeigt ist in Fig. 9c die Ausführung des Dichtungselements 50 mit einem axialen Gleitlager.

Die Kammer wird begrenzt durch die erste Seitenwand 32, die zweite Seitenwand 34, eine Kammerbodenwand 31, eine Kammerrückwand 33 und eine Kammerdeckenwand 38, wobei die Kammerbodenwand 31, und auch die Kammerdeckenwand 38, im bestimmungsgemäßen Gebrauch des Laborgeräts horizontal angeordnet ist.

Der Laborschüttler 1 weist ein Gehäuse 19 auf, in dem die Kammer 2 angeordnet ist. Die Kammeröffnung 2a der Kammer 2 ist bei geschlossener Schwenktüre 120, auch bezeichnet als Türblattelement 120, mittels Türdichtung 10a und Dichtungselementen 50, 50' etc. so abgedichtet, dass im Betrieb des Laborschüttlers 1 nur ein vernachlässigbarer Austausch von Gasen oder Wasserdampf zwischen Kammerinnenraum 3 und der Umgebung des Laborschüttlers stattfindet.

Die nachfolgend noch erläuterte Schwenktüreinrichtung und der Antrieb 20 des Laborschüttlers sind außerhalb der Kammer 2 angeordnet. Dadurch wird der Kammerinnenraum 3 effizient nutzbar, insbesondere werden die Antriebskomponenten während der Durchführung eines auf den Kammerinnraum angewandten Hochtemperatursterilisierungsverfahrens nicht mit erhitzt.

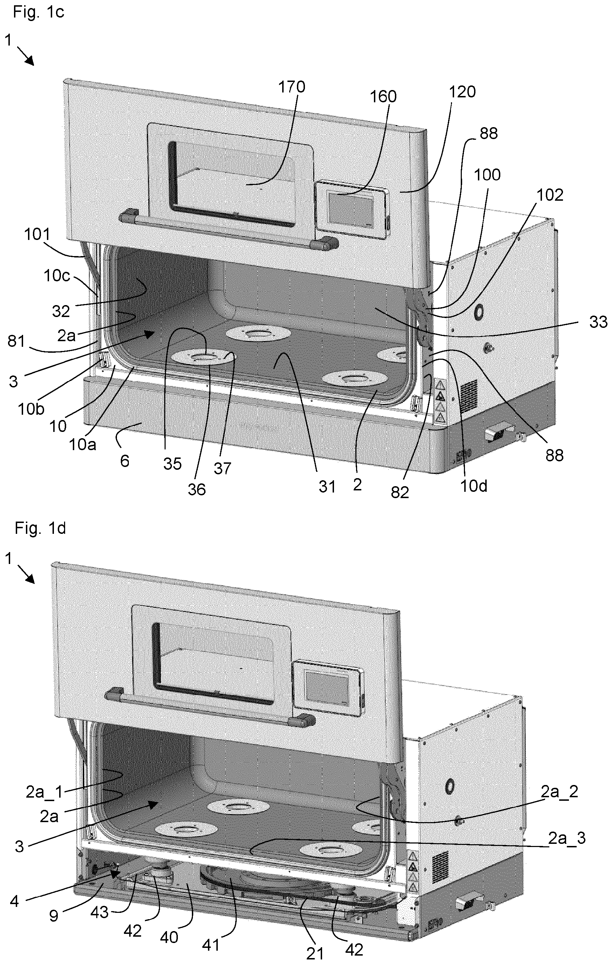

Insbesondere befindet sich ein Großteil des Antriebs im Vorrichtungsraum 4, auch bezeichnet als Antriebsraum 4, der unterhalb des Kammerbodens 31 angeordnet ist. Ein Teil des Antriebs 20 sowie weitere Komponenten befinden sich im Elektronikraum 5, der seitlich der Kammer angeordnet ist. Das Gehäuse 19 weist die Seitenwand 7 auf, eine weitere Seitenwand 7a, eine Rückwand, eine Frontblende 6, eine Deckenwand, eine Rückwandblende und Seitenblenden 8. Die Schwenktüre 120 ist durch eine Schwenkmechanik mit Gaszugfeder von der Frontwandebene 10 nach oben heraus schwenkbar, die geöffnete Position der Schwenktüre ist in Fig. 1c und 1d gezeigt.

In Fig. 1a ist die erste Position P1 der Schwenktüre 120 gezeigt, in der Schwenktüre 120 die Kammeröffnung 2a verschließt. Der Verschluss ist vollständig und dicht, was durch die umlaufende Silikondichtung 10a und durch eine Magneteinrichtung 180 erreicht wird. An der Innenseite der Schwenktüre 120 angebrachte Magneten (181, 182, 183, 184; siehe unten) haften in der Position P1 an der ferromagnetischen Frontwand des Laborgeräts oder an dort optional zusätzlich angebrachten Magneten, wobei die Silikondichtung 10a zusammengepresst wird. Die Magneten 181 und 182 können, bei ausreichender magnetischer Verschlusswirkung, auch weggelassen werden.

Fig. 1b zeigt den Laborschüttler 1, mit einer abgenommenen Seitenwand 7 und den seitlich der Kammer in einer Elektronikkammer 5 angeordneten Komponenten. Diese Komponenten beinhalten den Antrieb 20, hier ein BLDC-Motor, den dadurch angetriebenen Riemen 21, die Spannungsversorgungskomponenten 22 des Antriebs und der Heizeinrichtung zum Beheizen der Kammer, mit Lüfter 23, eine Elektronikplatine 24, welche insbesondere die Steuerungseinrichtung des Laborschüttlers beinhaltet. Diese ist insbesondere dazu programmiert, ein auf den Kammerinnraum 3 angewandtes Hochtemperatursterilisierungsverfahren, gemäß einem Aspekt der Erfindung, auszuführen. Sichtbar ist ebenso der Wasserverdampfer 25, mit dem Wasser verdampfbar und in den Kammerinnenraum einleitbar ist.

Fig. 1c zeigt den Laborschüttler 1, mit geöffneter Schwenktüre 120, wobei die Dichtungselemente, Verbindungselemente, Subplattform und Plattform sowie Probengefäße aus der Kammer entfernt sind und deshalb nicht dargestellt sind. Sichtbar ist die Kammer 2, die aus einstückig miteinander verbundenen Edelstahlwänden 31, 32, 33, 34, 38 (Kammer ist aus Edelstahl ist bevorzugt, Aluminium als Kammermaterial ist aber ebenfalls bevorzugt) gebildet wird, welche durch gewölbte Wandabschnitte integral gefertigt miteinander verbunden sind. Diese Kammerwände beinhalten die Kammerbodenwand 31, die Seitenwand 32, eine weitere Seitenwand 34, eine Deckenwand 38, und eine Rückwand 33. Die Kammerbodenwand 31 weist vier Bodenöffnungen 35 auf. Konzentrisch zu jeder Bodenöffnung ist jeweils ein kreisringförmiges Gleitflächenelement 36 aus Aluminium konzentrisch um die Bodenöffnung und auf der Kammerbodenwand 31 im Kammerinnenraum 3 montiert. Das Gleitflächenelement 36 weist an der Oberseite die (mit Toleranzen) parallel zur Kammerbodenwand angeordnete Gleitfläche 37 auf. Diese dient zur Gleitlagerung eines Dichtungselementes 50 (Fig. 9c, in Fig. 1c, d nicht gezeigt, siehe Fig. 2a, 2b), das zur axialen Gleitlagerung auf der Gleitfläche angeordnet wird.

Falls zu Wartungszwecken oder zum Zweck des Orbitwechsels das Schubladenelement 40 herausgezogen werden soll, muss beim Laborschüttlers in Fig. 1c die abnehmbare Frontblende 6 abgenommen werden. Man gelangt so zum in Fig. 1d dargestellten Zustand des Laborschüttlers.

Fig. 1d zeigt den Laborschüttler der Fig. 1c, mit abgenommener Frontblende 6, hinter der sich der, unterhalb der Kammer gelegene, Antriebsraum 4 befindet. Im Antriebsraum 4 ist ein Schubladenelement 40, hier eine Schubladenplatte 40, angeordnet, die mittels Schieneneinrichtung 43, die mit einer Basis 9 des Laborschüttlers 1 im Betrieb fest verbunden ist und zu Wartungszwecken nach vorne (in y-Richtung) aus dem Antriebsraum 4 herausziehbar ist. Auf der Schubladenplatte 40 ist die vom Riemen 21 angetriebene mit Exzenterkopplung 41a versehene, Antriebsscheibe 41 drehbar angeordnet, mittels der die als Übertragungsplatte 44 (hier nicht sichtbar) ausgebildete, und oberhalb (also in positiver z-Richtung) der Antriebsscheibe (Exzenterscheibe) 41 angeordnete Antriebskomponente in eine horizontale Schwenkbewegung versetzt wird. Vier bewegliche Sockelteile (Lagerelemente, Idler 42, insbesondere mit doppelt kugelgelagerter Exzenterwelle, wobei ein oberes Kugellager elastisch gelagert ist), sind auf der Schubladenplatte 40 montiert, welche einerseits die Übertragungsplatte 44 tragen und deren horizontal beweglichen Lagermechaniken andererseits der von der Exzenterscheibe 41 erzeugten horizontalen Schüttelbewegung folgen.

Die Schubladeneinrichtung beinhaltet das Schubladenelement 40 und die Schienen 43, auf denen das Schubladenelement 40 beweglich angeordnet ist, hier für eine gleitende Translationsbewegung zwischen einer ersten und einer zweiten Position. Das Schubladenelement 40 kann, im Ausführungsbeispiel des Laborschüttlers 1, nach Lösen einiger Schrauben, welche die Schubladenplatte 40 im Betriebszustand fest mit der Gerätebasis 9 verbinden, in Richtung nach vorne aus dem Antriebsraum 4 herausgezogen werden. Dabei wird das Schubladenelement 40 aus einer ersten Position, die in Fig. 1d gezeigt ist, in der das Schubladenelement 40 im Antriebsraum angeordnet ist, in eine zweite Position bewegt, in der das mindestens eine Schubladenelement 40 aus dem Antriebsraum 4 ausgefahren ist. Entsprechend kann das Schubladenelement 40 aus der zweiten Position wieder zurück in die erste Position bewegt werden, insbesondere nachdem vom Wartungspersonal die gewünschte Maßnahme an der Antriebsvorrichtung 41, 42, 21 ausgeführt wurde.

"Nach vorne" bezeichnet die senkrecht von der Türe 10 des Laborschüttlers nach außen weisende Richtung (y-Richtung, wie in Fig. 1a gezeigt).

In Fig. 1d ist die zweite Position P2 der Schwenktüre 120, in der diese vollständig von der Kammeröffnung entfernt ist und diese ungehindert von vorne zugänglich ist. Die Kammeröffnung 2a wird begrenzt durch einen ersten Seitenrand 2a_1 und einen, diesem gegenüberliegend, zweiten Seitenrand 2a_2, sowie einen Unterrand 2a_3 und, diesem gegenüberliegend, einen Oberrand 2a_4, wobei der erste und zweite Seitenrand senkrecht zu dem Oberrand und dem Unterrand verlaufen. Im bestimmungsgemäßen Gebrauch des Laborgeräts sind der Unterrand 2a_3 und der Oberrand 2a_4 horizontal angeordnet.

Die Figuren 2a bis 2f zeigen verschiedene Positionen der Schwenktüreinrichtung 100 bzw. der Schwenktüre 120. Fig. 1a zeigt die Schwenktüre 120 in der ersten Position P1, in der diese die Kammeröffnung 2a vollständig verschließt. Fig. 2f zeigt die zweite Position P2 der Schwenktüre 120, in der diese vollständig von der Kammeröffnung weggeschwenkt und maximal entfernt ist und die Kammer ungehindert von vorne zugänglich ist. Die Figuren 2b bis 2e zeigen Positionen der Schwenktüre zwischen der ersten Position P1 und der zweiten Position P2, wenn die Schwenktüre 120 geöffnet wird und dabei, geführt durch die Schwenktüreinrichtung 100, nach oben gleitet. Die Schwenktüre 120 wird deshalb auch als Türe des nach-oben-gleitenden Typs (Englisch: "slide-up door") bezeichnet.

Fig. 2b bis 2e zeigen geöffnete Positionen der Schwenktüre, in der die Kammeröffnung geöffnet ist, aber die Kammertüre noch nicht vollständig von der Kammeröffnung 2a weggeschwenkt ist, die Kammer somit noch nicht für ein Beladen oder Entladen der Kammer vollständig zugänglich ist.

Fig. 2a zeigt insbesondere die Trägereinrichtung 80. Diese weist als erstes und zweites Trägerteil zwei seitliche, sich gegenüberliegende Rahmenseitenwände 89, und 88 (siehe auch Fig. 4b) auf, die aus Edelstahlblech bestehen und mit der Gerätebasis 9 fest verbunden sind. Das erste Trägerteil 89 und das zweite Trägerteil 88 (siehe Fig. 1c) weisen vertikal angeordnete Trägerstreben 81, 82, 83, 85, 87, aus profiliertem Stahlblech auf, die als integrale Bestandteile der Rahmenseitenwände 89 und 88 gebildet sind. Gemeinsam mit anderen vertikal angeordneten Trägerstreben, insbesondere der Trägerstrebe 83, und horizontal angeordneten Trägerstreben bilden die Rahmenseitenteile 89, 88 einen Trägerrahmen 80, der ein Teil der Trägereinrichtung ist oder diese bildet.

Fig. 2b zeigt eine Position der Schwenktüre 120, kurz nach dem Öffnen der Kammeröffnung 2a, wenn der Benutzer den mit der Schwenktüre 120 fest verbundenen Handgriff 11 zum Öffnen nach vorne gezogen hat. Die dabei aufzubringende Kraft beinhaltet im Wesentlichen die Haltekraft zum Lösen der vorliegend vier - an der Innenseite der Schwenktüre angebrachten - Permanentmagneten, die in der Position P1 auf der ferromagnetischen Frontwand 10 des Laborgeräts haften, bzw. zwischen dort angebrachten Permanentmagneten 10b berührungsfrei gehalten werden können, die in Fig. 1c gezeigt sind und die in den Figuren 2b bis 2e nicht gezeigt sind.

Zum vollständigen Öffnen der Schwenktüre zieht der Benutzer den Handgriff 11 weiter nach vorne (Figuren 2c bis 2e). Dadurch wird die Schwenktüre, geführt durch die Schwenktüreinrichtung 100, nach oben bewegt. Diese Bewegung erfolgt im Wesentlichen ohne Kraftaufwand seitens des Benutzers, da die in einem Federsystem 150 der Schwenktüreinrichtung gespeicherte Energie zum Nach-oben-Schwenken der Schwenktüre 120 verwendet wird. Das Federsystem 150 beinhaltet vorliegend zwei Gasfedern, hier Gaszugfedern S151, 152. Bevorzugt ist generell, dass das Federsystem mindestens eine Feder beinhaltet. Diese kann eine Schraubenfeder, insbesondere eine Druckfeder oder eine Zugfeder sein, oder eine Gasfeder, insbesondere eine Gasdruckfeder oder eine Gaszugfeder sein.

Wie in den Figuren 1c sowie 2b bis 2e und 5a, 5b zu sehen ist, weist der Laborschüttler 1 eine Schwenktüreinrichtung 100 zum Verschließen der Kammeröffnung 2a auf. Zur Schwenktüreinrichtung 100 gehört das schwenkbares Türblattelement 120. Die Schwenktüreinrichtung 100 beinhaltet vorliegend zudem, benachbart dem ersten Seitenrand 2a_1 der Kammeröffnung 2a angeordnet, einen ersten, unteren Schwenkarm 101 und einen ersten, oberen Schwenkarm 103. Die Schwenktüreinrichtung 100 beinhaltet vorliegend zudem, benachbart dem zweiten Seitenrand 2a_2 der Kammeröffnung 2a angeordnet, einen zweiten, unteren Schwenkarm 102 und einen zweiten, oberen Schwenkarm 104. Die ersten Schwenkarme 101, 103 liegen jeweils den zweiten Schwenkarmen 102, 104 gegenüber und sind um eine Distanz voneinander beabstandet, welche größer ist als die maximale Breite der Kammer 2 und welche kleiner ist als die Gesamtbreite des Laborgeräts 1.

Das Laborgerät weist, wie in Fig. 2c oder Fig. 1c gezeigt ist, eine erste, schlitzförmige Aussparung 10c auf, in welche der mindestens eine erste Schwenkarm 101, 103 eingreift. Das Laborgerät weist zudem eine zweite schlitzförmige Aussparung 10d auf, in welche der mindestens eine zweite Schwenkarm 102, 104 eingreift. Die Aussparung mündet jeweils in einer Öffnung der Frontwand 10 des Laborgeräts. Der erste untere Schwenkarm 101 und der erste obere Schwenkarm 103 sind derart schwenkbar an der Trägereinrichtung 80 angeordnet, dass sie während ihrer jeweiligen Schwenkbewegung stets koplanar sind, also in derselben ersten Ebene liegen. Der zweite untere Schwenkarm 102 und der zweite obere Schwenkarm 104 sind derart schwenkbar an der Trägereinrichtung angeordnet, dass sie während ihrer jeweiligen Schwenkbewegung stets koplanar sind, also in derselben zweiten Ebene liegen. Die erste und die zweite Ebene sind parallel und zueinander beabstandet. Durch diese Anordnung können die genannten Aussparungen eine plattenförmige Form aufweisen, was eine kompakte Konstruktion der Schwenktüreinrichtung und des Laborgeräts ergibt.

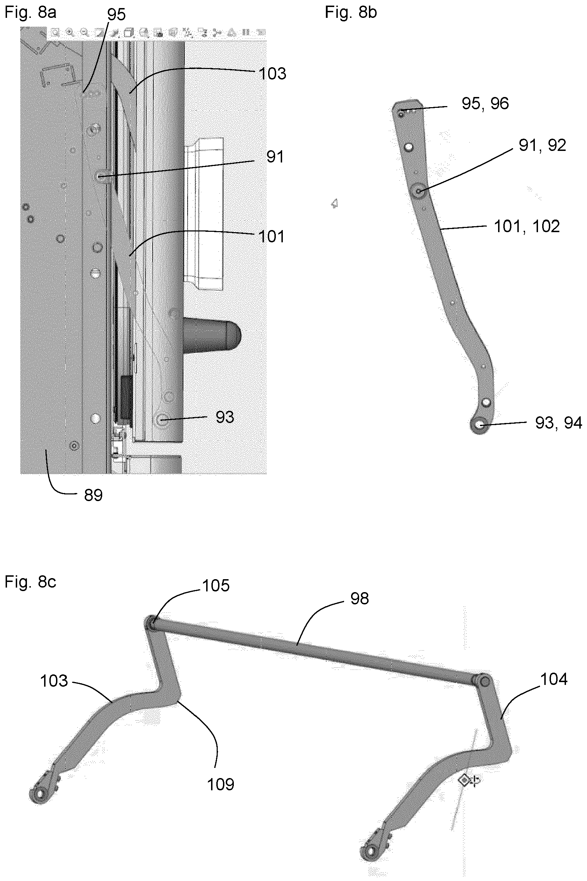

Der erste obere Schwenkarm 103 und der zweite obere Schwenkarm 104 sind über die Kopplungsstange 98 fest miteinander verbunden. Diese Verbindung durch die Kopplungsstange 98 ist in Fig. 8c gezeigt. Die Anordnung aus oberem erstem Schwenkarm 103, Kopplungsstange 98 und oberem zweitem Schwenkarm 104 ist ein starres Gebilde, wobei die genannten Bestandteile aus Edelstahl bestehen. Deshalb bewegt sich der erste Schwenkarm, wenn sich der zweite Schwenkarm bewegt, und umgekehrt. Die Bewegungen diese Schwenkarme sind synchronisiert. Die Kopplungsstange 98 ist außerhalb der Kammer angeordnet. Die Kopplungsstange 98 ist drehbar an der Trägereinrichtung 80 gelagert. Dazu ist eine Hülse 105 vorgesehen, die oberhalb der Kammer 2 fest mit der Trägereinrichtung 80 verbunden ist.

Fig. 3a bis Fig. 3c zeigen insbesondere, dass die Schwenkarme an Schwenklagern gelagert sind, die hier als Gleitlager ausgeführt sind. Jeder Schwenkarm 101, 102, 103, 104 ist einerseits unmittelbar (Schwenkarme 101, 102) oder mittelbar (Schwenkarme 103, 104, via der Kopplungsstange 98) an der Trägereinrichtung 80 gelagert. Der erste untere Schwenkarm 101 ist mittels des Schwenklagers 91 am ersten Trägerteil 81 schwenkbar gelagert. Das erste Schwenklager 91 beinhaltet einen Stift, der in eine Bohrung des ersten unteren Schwenkarms 101 und eine Bohrung des ersten Trägerteils 81 eingreift.

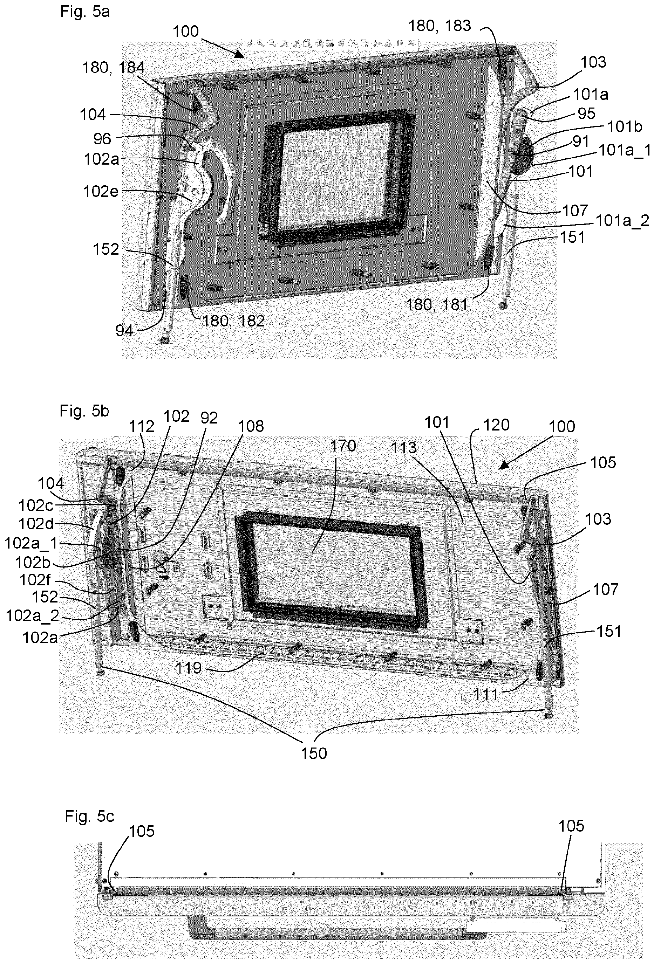

Der erste untere Schwenkarm 101 ist zudem mittels eines Schwenklagers 93 am Türblattelement 120 schwenkbar gelagert. Dort befindet sich das entsprechende Schwenklager 93 an einem Profilblech 107 (siehe Figur 5a), das fest mit der Innenseite des Türblattelements 120 verbunden ist und das zur Ebene des Türblattelements 120 senkrecht und von oben nach unten verläuft.

Der erste untere Schwenkarm 101 weist zudem ein drittes Schwenklager 95 auf, an dem ein Ende der Gasfeder 151, insbesondere deren Kolbenende, schwenkbar gelagert ist. Das andere Ende der Gasfeder 151 ist in einem unteren Bereich des ersten Trägerteils 81 schwenkbar gelagert.

Die Schwenkachse des Schwenklagers 95 liegt näher an der Schwenkachse 91 als die Schwenkachse 93. Dadurch wird eine Hebelwirkung erzielt, um im Zusammenwirken mit der Gasfeder 151 die geeignete Kraft zum Öffnen der Schwenktüreinrichtung 100 zu erzielen, mit der zum Beispiel ein Benutzer durch Auslenken nach oben des Griffs 11 die Schwenktüre 120 öffnet. Insbesondere ist die Kraft zum Spannen der Gasfeder durch die Hebelwirkung reduziert, wenn zum Beispiel ein Benutzer die Schwenktüre 120 durch Auslenken des Handgriffs 11 nach unten wieder schließt.

Die Schwenkachsen aller drei Schwenklager 91, 93 und 95 sind parallel zueinander.

Der zweite untere Schwenkarm 102 ist hier spiegelsymmetrisch zum ersten Schwenkarm 101 gestaltet (siehe Fig. 8b):
Der zweite untere Schwenkarm 102 ist mittels des Schwenklagers 92 (siehe Fig. 5b) am zweiten Trägerteil 82 schwenkbar gelagert. Dazu beinhaltet das zweite Schwenklager 92 ebenfalls einen Stift, der in eine Bohrung 92 des zweiten unteren Schwenkarms 102 und eine Bohrung des zweiten Trägerteils 82 eingreift.

Der zweite untere Schwenkarm 102 ist mittels eines weiteren Schwenklagers 94 am Türblattelement 120 schwenkbar gelagert. Dort befindet sich das entsprechende Schwenklager 94 an einem, dem Profilblech 107 gegenüberliegenden, Profilblech 108 (siehe Figur 5b), das fest mit der Innenseite des Türblattelements 120 verbunden ist und das zur Ebene des Türblattelements 120 senkrecht und von oben nach unten verläuft. Die planaren und parallelen Profilbleche 107, 108 haben somit im Wesentlichen denselben Abstand wie die einander zugewandten Seiten der Schwenkarme 101 und 102.

Der zweite untere Schwenkarm 102 weist zudem ein drittes Schwenklager 96 auf, an dem ein Ende der Gasfeder 152, insbesondere deren Kolbenende, schwenkbar gelagert ist. Das andere Ende der Gasfeder 152 ist in einem unteren Bereich des zweiten Trägerteils 82 schwenkbar gelagert.

Die Schwenkachsen aller drei Schwenklager 92, 94 und 96 sind parallel zueinander.

Die Schwenkachse des Schwenklagers 96 liegt näher an der Schwenkachse 92 als die Schwenkachse 94. Dadurch wird ebenfalls die Hebelwirkung erzielt, um im Zusammenwirken mit der zweiten Gasfeder 152 die geeignete Kraft zum Öffnen der Schwenktüreinrichtung 100 zu erzielen, mit der zum Beispiel ein Benutzer durch Auslenken nach oben des Griffs 11 die Schwenktüre 120 öffnet. Insbesondere ist die Kraft zum Spannen der Gasfeder durch die Hebelwirkung reduziert, wenn zum Beispiel ein Benutzer die Schwenktüre 120 durch Auslenken des Handgriffs 11 nach unten wieder schließt.

Die gewünschte Kraft zum Öffnen und Schließen der Schwenktüre entsteht somit insbesondere durch die Hebelwirkung beider unterer Schwenkarme 101, 102 als auch durch die gemeinsame Wirkung der beiden Gasfedern 151 und 152.

Die Schwenkarme 101, 102, 103 und 104 sind planare Bauteile. Der untere erste Schwenkarm 101 und der untere zweite Schwenkarm 102 sind leistenförmige Bauteile, deren Länge und Breite mehrfach größer sind als ihre Dicke. Die relativ geringe Dicke ist unschädlich hinsichtlich der mechanischen Stabilität der Schwenktüreinrichtung, da die Schwenkbewegung in der durch die Länge und Breite aufgespannte Hauptebene der Leiste lokalisiert ist. Dadurch wird eine Last bzw. ein Biegemoment senkrecht zu der Hauptebene vermieden. Auch der obere erste Schwenkarm 103 und der obere zweite Schwenkarm 104 sind leistenförmige planare Bauteile, deren Länge und Breite mehrfach größer sind als ihre Dicke. Sie weisen einen Knick auf (siehe Fig. 8c), der dazu dient, ein Kontaktieren der beiden unteren Schwenkarme und ein Kontaktieren der Schwenktüre 120 während des Öffnens oder Schließens der Schwenktüre 120 zu vermeiden. Dies lässt sich anhand der Figuren 3a bis 3c nachvollziehen.

Die Trägereinrichtung 80 weist die beiden gegenüberliegenden Rahmenseitenwände 88, 89 auf, zwischen denen die Kammer 2 angeordnet und an denen sie, zusätzlich zur Lagerung an der Gerätebasis 9, gelagert ist.. Der Zwischenraum zwischen der Rahmenseitenwand 89 und der ersten Kammerseitenwand 32 ist durch ein thermisches Isoliermaterial, insbesondere PU-Schaum, ausgefüllt, ebenso der Zwischenraum zwischen der Rahmenseitenwand 88 und der zweiten Kammerseitenwand 34.

Eine weitere Funktion der unteren Schwenkarme 101, 102 wird in Bezug auf die Figuren 5a und 5b erläutert. Der erste untere Schwenkarm 101 ist an seiner kammerabgewandten Seite mit einem plattenförmigen Bauteil 101a versehen. Dieses kann aus Aluminium gefertigt sein oder aus einem anderen Metall oder aus Kunststoff. Es erstreckt sich entlang der Längsrichtung des unteren ersten Schwenkarms 101 und ist mit diesem fest verbunden. Das plattenförmigen Bauteil 101a weist größere Abschnitte 101a_1 und 101a_2 auf, mit denen das plattenförmige Bauteil 101a über die Fläche des Schwenkarms 101 hinausragt. Im Abschnitt 101a_1 weist das plattenförmige Bauteil 101a an seiner der Kammer zugewandten Seite einen Gleitstein 101b auf. Dieser ist dazu eingerichtet und dazu angeordnet, beim Öffnen und Schließen der Schwenktüre an der kammerabgewandten Fläche der Rahmenseitenwand 89 entlang zu gleiten. Letztere erfüllt somit auch die Funktion einer Gleitfläche. Der Gleitstein 101b kann zum Beispiel aus PTFE oder PEEK bestehen.

Analog ist der zweite untere Schwenkarm 102 ist an seiner kammerabgewandten Seite mit einem plattenförmigen Bauteil 102a versehen. Dieses kann aus Aluminium gefertigt sein oder aus einem anderen Metall oder aus Kunststoff. Es erstreckt sich entlang der Längsrichtung des unteren zweiten Schwenkarms 102 und ist mit diesem fest verbunden. Das plattenförmigen Bauteil 102a weist größere Abschnitte 102a_1 und 102a_2 auf, mit denen das plattenförmige Bauteil 102a über die Fläche des Schwenkarms 102 hinausragt. Im Abschnitt 102a_1 weist das plattenförmige Bauteil 102a an seiner der Kammer zugewandten Seite einen Gleitstein 102b auf. Dieser ist dazu eingerichtet und dazu angeordnet, beim Öffnen und Schließen der Schwenktüre an der kammerabgewandten Fläche der Rahmenseitenwand 88 entlang zu gleiten. Letztere erfüllt somit auch die Funktion einer Gleitfläche. Der Gleitstein 102b kann zum Beispiel aus PTFE oder PEEK bestehen.

Eine in Figur 5a und 5b gezeigte gebogene Gleitkulisse 102d ist mit der Rahmenseitenwand 88 verschraubt. Beim Öffnen und Schließen der Schwenktüre 120 gleitet eine Stirnseite 102c des unteren zweiten Schwenkarms 102 an der gebogenen Innenseite der Gleitkulisse 102d entlang. Dadurch wird die Schwenkbewegung stabilisiert.

Durch die Anlage des Gleitsteins 101b an der Rahmenseitenwand 89 und der Anlage des Gleitsteins 102b an der Rahmenseitenwand 88 wird die Schwenkbewegung weiter stabilisiert.

Parallel zu dem plattenförmigen Bauteil 102a weist der Schwenkarm 102 ein weiteres plattenförmigen Bauteil 102e auf, das analog zum plattenförmigen Bauteil 102a geformt ist und zu diesem in einem Abstand parallel angeordnet ist. Die Befestigung des plattenförmigen Bauteil 102e am plattenförmigen Bauteil 102a erfolgt durch Verbindungsstifte 102f, die zwischen diesen Bauteilen angeordnet sind. Die Druckfeder ist an der kammerabgewandten Seite des plattenförmigen Bauteils 102e an der Position der Schwenkachse 96 angebracht. Zwischen den plattenförmigen Bauteilen 102a und 102e können elektrische Kabel verlaufen, welche die Benutzerschnittstelle 160, die an der Frontseite der Schwenktüre 120 angebracht ist, mit einer elektronischen Steuereinrichtung des Laborgeräts verbinden, zum Zwecke der Spannungsversorgung der Benutzerschnittstelle 160 und einer Heizeinrichtung der Schwenktüre, und des Datenaustausches, insbesondere per Ethernet, insbesondere während der Schwenkbewegung der Schwenktüre.

Figur 5b zeigt, dass das Türblattelement 120 ein mehrwandiges und thermisch isolierendes Sichtfenster 170 aufweist. Die planaren und parallelen Profilbleche 107, 108 dienen ebenfalls als Versteifungselemente des Türblattelements 120 und sind fest mit der Innenseite 113 einer Frontwand des Türblattelements 120 verbunden. Die planaren und parallelen Profilbleche 107, 108 tragen oder münden in planare Plattenabschnitte 111, 112, bzw. Blechabschnitte 111, 112, welche parallel zur Frontwand 113 verlaufen. Die planaren Plattenabschnitte 111, 112 bilden eine geöffnete Innenwand des Türblattelements 120. Die Öffnung der Innenwand des Türblattelements 120 ist insbesondere größer als die Kammeröffnung. In Figur 5a, 5b nicht gezeigt ist die thermische Isolierung aus Glas- oder Steinwolle, welche die Schwenktüre zu einem thermischen Isolationselement 125 macht, das die Kammeröffnung 2a in Position P1 der Schenktüre 120 effektiv thermisch von der Umgebung isoliert. Dies ist in Figur 9c gezeigt. An der Innenwand 111, 112 der Schwenktüre 120 sind vier Magneten 181, 182, 183, 184 einer Magneteinrichtung 180 befestigt, wobei insbesondere die Magneten 181, 182 optional sind. Diese verbinden die Schwenktüre in der Position P1 mit einer aus magnetischem Blech gefertigten Frontwand oder mit dort angebrachten Paaren 10b aus Permanentmagneten, die auf der Frontwand 10 befestigt sind und zwischen die die Magneten 181, 182, 183, 184 berührungsfrei eintauchen und die magnetische Verschlusskraft etablieren.

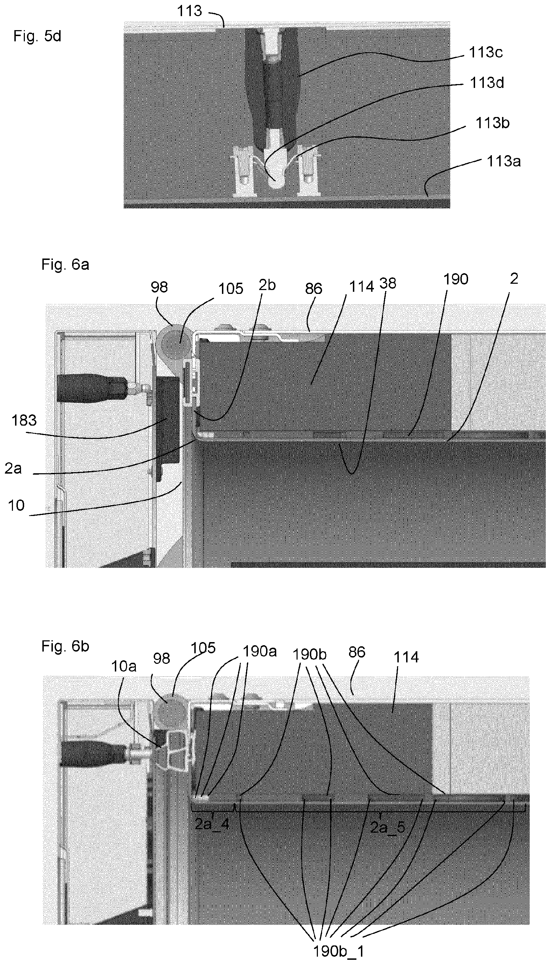

Fig. 5d zeigt ein Detail eines Querschnitts senkrecht zum Türblattelement 120, durch einen der 12 Haltebolzen 113c, die an der Innenseite der Außenwand 113 des Türblattelements 120 fest montiert sind. Diese Haltebolzen 113c weisen jeweils einen Kugelkopf 113d auf, die in jeweils an der Innenwand 113a des Türblattelements 120 vorgesehenen Rasteinrichtungen 113b befestigt sind. Die Innenwand 113a weist, im Türinneren gelegen, Heizwendel auf.

Die Magneten 181, 182, 183, 184 sind vorzugsweise positionsfeste Permanentmagneten, können aber auch positionsvariable, zum Beispiel drehbare Permanentmagneten sein oder können Elektromagneten sein. Ein Magnet 181, 182, 183, 184 kann mehrere Magnetelemente beinhalten, die insbesondere zueinander beweglich sein können, um in einer ersten Stellung ein starkes gemeinsames Magnetfeld zu bilden, welches die Schwenktüre in Position P1 mit der Frontwand 10 verbindet, und in einer zweiten Stellung ein schwaches gemeinsames Magnetfeld zu bilden, welches ein einfaches Lösen der in Position P1 mit der Frontwand 10 verbundenen Schwenktüre ermöglicht.

Das Türblattelement weist hier mehrere Versteifungsprofile bzw. Versteifungsstreben 107, 108, 119 auf.

Figur 6a zeigt anhand eines Schnitts durch das Laborgerät senkrecht zum Türblattelement 120 und entlang der in Fig. 2a gezeigten Linie A-A, wie der Magnet 183 beinahe die Frontwand 10 kontaktiert. Figur 6b zeigt denselben Schnitt, bei dem aber auch die Silikondichtung 10a gezeigt ist, welche um die Kammeröffnung 2a herumläuft und die den Blick auf den Magneten 183 verdeckt.

Fig. 6a und 6b zeigen zudem den mit thermischem Isolierschaum 114 gefüllten Zwischenraum zwischen der Kammerdeckenwand 38 und der Deckenblechwand 86 des Trägerrahmens 80. Die thermische Isolation der Kammer wird weiter dadurch verbessert, dass der Kammerflansch 2b, der einstückig mit der Kammer 2 (aus Edelstahl) verbunden ist, die Trägereinrichtung 80 bzw. deren Blechwände 86 nicht direkt berührt, sondern über eine Kunststoffabstandsleiste 115 mittelbar mit der Trägereinrichtung 80 verbunden ist. Dadurch wird die unerwünschte Wärmeübertragung zwischen Trägereinrichtung 80 und Kammer 2 auf ein Minimum beschränkt. Kunststoffabstandshalter sind ebenfalls in anderen Bereichen im Innenraum des Laborgeräts zwischen Trägereinrichtung 80 und Kammer 2 vorgesehen, um die Kammer abzustützen.

Fig. 6b zeigt auch die Heizeinrichtung 190, die zum Beheizen der Kammer vorgesehen ist, die einen Heizwendel 190a aufweist, der an der Außenseite der mindestens einen Kammerwand angeordnet ist, wobei die mindestens eine Kammerwand mindestens einen ersten Flächenbereich aufweist, in dem die von dem mindestens ein Heizwendel abgegebene Heizleistung größer ist als in einem zweiten Flächenbereich, insbesondere indem der mindestens ein Heizwendel im ersten Bereich mit einer höheren Flächendichte verlegt ist. Der erste Flächenbereich ist jener, der an der Kante 2a_4 als Randbereich der Kammer verläuft. Dort sind drei Heizdrähte 190a unmittelbar nebeneinander verlegt, insbesondere im Kontakt zueinander, während im zweiten Flächenbereich, hier zum Beispiel im zentralen Bereich 2a_5 der Kammerdeckenwand, von dem/den Heizdrähten 190b eine geringere Heizleistung pro Fläche abgegeben wird.

Durch die Art der Darstellung sind im Bereich 2a_5 jeweils Stücke von horizontalen Stegen 190b sichtbar, es handelt sich um eine gemischte Querschnitts- und Seitenansicht, bei der auch die tiefer liegenden Kurven der Heizwendel sichtbar sind. Der jeweilige Drahtquerschnitt ist durch den quadratischen Querschnitt 190b_1 repräsentiert, der sich am Ende eines horizontalen Steges sichtbar ist. Die Flächengröße der Bezugsfläche im Parameter "Flächendichte" (der vom Draht belegte Oberflächenanteil A_H geteilt durch die Bezugsflächengröße A) bezieht sich hier auf die durchschnittliche, von einem Heizwendel 190b umrandete Fläche, multipliziert mit einem Faktor f gewählt aus {1, 2, ..., 10}, vorzugsweise f=2, als Bezugsflächengröße. Ein Heizwendel wird hierbei als ein Draht 190c verstanden, der als Schlaufe verlegt ist, also zwei gerade parallele Stücke aufweist, die durch einen 180°-Kurvenabschnitt verbunden sind, siehe Fig. 9c. Alternativ kann die Bezugsflächengröße auch gewählt sein als ein Anteil der gesamten Kammerwandaußenfläche, die durch zwei Seitenwände, Bodenwand, Deckenwand und Rückwand, sowie den jeweiligen gekrümmten Verbindungsabschnitten gegeben ist. Der Anteil kann gewählt sein aus den bevorzugten Anteilen {1/30; 1/20; 1/10; 1/5}.

Durch die hohe Flächendichte des Heizdrahtes an der Kammeröffnung 2a_4 lassen sich Wärmeverluste kompensieren, die durch die Lage der Drähte an der Kammeröffnung bedingt sind. Die Temperaturverteilung entlang der Kammer wird durch die Variation der Flächendichte in den Bereichen 2a_4 und 2a_5 insgesamt homogener und das Risiko für Kondensatbildung wird reduziert. Auch im Bereich der gekrümmten Übergangsflächen zwischen den Kammerwänden (z.B. zwischen Rückwand und Deckenwand, Rückwand und Seitenwänden und Rückwand und Bodenwand) liegt eine höhere Flächendichte vor als im zentralen Bereich 2a_5, was ebenfalls zur Verbesserung der Temperaturhomogenität beiträgt.

Fig. 9a zeigt die Rückseite des Inkubators, mit abgenommener Gehäuseaußenwand, und mit entfernter Isolationsmaterialschicht 116, die im Zwischenraum zwischen der Kammerrückwand und der Gehäuseaußenwand angeordnet ist. Die Isolationsmaterialschicht 116 (Pu-Schaum) ist aber in Figur 9b gezeigt.

Figur 9c zeigt einen vertikalen Schnitt durch das Laborgerät 1 senkrecht zur Schwenktüre 120. Die Plattformeinrichtung 190 sitzt auf Kopplungsstangen, welche vertikal durch die Kammerbodenwand 31 nach oben ragen und die im Antriebsraum 4 vorgesehene Übertragungsplatte 44 mit der Plattformeinrichtung 190 verbinden, welche Probengefäße, wie die gezeigten Erlenmeyerkolben, trägt. Die Übertragungsplatte 44 ist mit dem Antrieb gekoppelt und wird im Betrieb des Inkubationsschüttlers 1 geschüttelt.

Die Isolationsmaterialplatten 114, 117, 116, 118, 125 bilden bei geschlossener Kammeröffnung 2a eine Isolationseinhausung um einen Innenraum, in dem sich die Kammer 2 befindet. In der Kammer 2 befindet sich notwendiger Weise die Plattformeinrichtung 190, es befinden sich dort aber weder Komponenten der Schwenktüreinrichtung 100 noch solche der Antriebseinrichtung. Dadurch wird das Kammervolumen optimal nutzbar.

## Patentansprüche

1. Laborgerät (1) zur Behandlung von in Probengefäßen (130) enthaltenen flüssigen Laborproben, insbesondere Inkubationsschüttler,
mit einer Kammer (2), die eine Kammeröffnung (2a) zum Einstellen und Herausnehmen der Probengefäße (130) in die Kammer sowie eine erste Seitenwand (32) und eine zweite Seitenwand (34) aufweist,
mit mindestens einer Schwenktüreinrichtung (100) zum Verschließen der Kammeröffnung (2a), die ein schwenkbares Türblattelement (120) aufweist, das zwischen einer ersten Position (P1), in der die Kammeröffnung (2a) durch das Türblattelement (120) verschlossen ist, und einer zweiten Position (P2) bewegbar ist, in der das Türblattelement (120) vollständig von der Kammeröffnung weggeschwenkt und entfernt ist und diese geöffnet ist, und
mit einer Trägereinrichtung (80) zum Tragen der Schwenktüreinrichtung (100),
**dadurch gekennzeichnet, dass**
die Schwenktüreinrichtung (100) mindestens einen ersten Schwenkarm (101; 103) und einen zweiten Schwenkarm (102; 104) aufweist, welche in der ersten Position des Türblattelements (120) außerhalb der Kammer (2) angeordnet sind und welche das Türblattelement (120) schwenkbar mit der Trägereinrichtung (80) verbinden.

2. Laborgerät gemäß Anspruch 1, wobei die Kammer (2) eine erste Seitenwand (32) und, dieser gegenüberliegend, eine zweite Seitenwand (34) aufweist, wobei der mindestens eine erste Schwenkarm (101) und der der mindestens eine zweite Schwenkarm (102) einander gegenüberliegend und parallel zueinander angeordnet sind und insbesondere parallel zu der ersten Seitenwand (32) und der zweiten Seitenwand (34) angeordnet sind.

3. Laborgerät gemäß einem der vorangehenden Ansprüche, das eine Basis (9), und damit verbunden, die Trägereinrichtung (80), insbesondere eine Rahmeneinrichtung (80), aufweist, welche Bestandteile des Laborgeräts tragen,
wobei die Trägereinrichtung ein erstes Trägerteil (89) mit einem ersten Schwenklager (91) aufweist, an dem der mindestens eine erste Schwenkarm (101) schwenkbar gelagert ist, und die Trägereinrichtung ein dem ersten Trägerteil (89) gegenüberliegendes zweites Trägerteil (88) mit einem zweiten Schwenklager (92) aufweist, aufweist, an dem der mindestens eine zweite Schwenkarm (102) schwenkbar gelagert ist.

4. Laborgerät gemäß einem der vorangehenden Ansprüche, wobei die Trägereinrichtung (80) eine erste Rahmenseitenwand (89) aufweist, und wobei der mindestens eine erste Schwenkarm (101; 103) an einer der Kammer (2) abgewandten Außenseite der Rahmenseitenwand (89) angeordnet ist, und
wobei die Trägereinrichtung (80) eine zweite Rahmenseitenwand (88) aufweist, und wobei der mindestens eine zweite Schwenkarm (102; 104) an einer der Kammer (2) abgewandten Außenseite der Rahmenseitenwand (88) angeordnet ist,
wobei insbesondere die erste Rahmenseitenwand (89) ein erstes Trägerteil (81) ist und insbesondere die zweite Rahmenseitenwand (88) ein zweites Trägerteil (82) ist.

5. Laborgerät gemäß Anspruch 4, wobei der Zwischenraum zwischen einer ersten Kammerseitenwand (32) und der ersten Rahmenseitenwand (89) der Trägereinrichtung mit einem thermischen Isolationsmaterial (125) gefüllt ist, und
wobei der Zwischenraum zwischen einer zweiten Kammerseitenwand (34) und der zweiten Rahmenseitenwand (88) der Trägereinrichtung mit einem thermischen Isolationsmaterial (125) gefüllt ist.

6. Laborgerät gemäß einem der vorangehenden Ansprüche, das ein Federsystem (150) aufweist, welches das Öffnen des Türblattelements (120) unterstützt, wobei das Federsystem insbesondere durch das Schließen des Türblattelements (120) spannbar ist.

7. Laborgerät gemäß Anspruch 6, das mindestens ein erstes Federelement (151) aufweist, insbesondere eine Gasfeder (151), das an seinem ersten Ende mit einem ersten Schwenkarm (101) und mit seinem zweiten Ende mit der Trägereinrichtung (80) verbunden ist, und das mindestens ein zweites Federelement (152) aufweist, insbesondere eine Gasfeder (152), das an seinem ersten Ende mit einem zweiten Schwenkarm (102) und mit seinem zweiten Ende mit der Trägereinrichtung (80) verbunden ist.

8. Laborgerät gemäß einem der vorangehenden Ansprüche, wobei ein erster Schwenkarm (101) mittels einem ersten Lagerelement (91) an der Trägereinrichtung (80) um eine erste Schwenkachse schwenkbar gelagert ist, mittels einem zweiten Lagerelement (93) an dem Türblattelement (120) um eine zweite Schwenkachse schwenkbar gelagert ist, und mittels einem dritten Lagerelement (95) an einem Federelement (151) um eine dritte Schwenkachse schwenkbar gelagert ist, wobei die erste, zweite und dritte Schwenkachse parallel zueinander verlaufen, und
wobei insbesondere ein zweiter Schwenkarm (102) mittels einem ersten Lagerelement (92) an der Trägereinrichtung (80) um eine erste Schwenkachse schwenkbar gelagert ist, mittels einem zweiten Lagerelement (94) an dem Türblattelement (120) um eine zweite Schwenkachse schwenkbar gelagert ist, und mittels einem dritten Lagerelement (96) an einem Federelement (152) um eine dritte Schwenkachse schwenkbar gelagert ist, wobei die erste, zweite und dritte Schwenkachse parallel zueinander verlaufen,
wobei insbesondere der Abstand zwischen dem ersten Lagerelement (91; 92) und dem zweiten Lagerelement (93; 94) größer ist, insbesondere 2 bis 4 mal größer ist, als der Abstand zwischen dem ersten Lagerelement (91; 92) und dem dritten Lagerelement (95; 96).

9. Laborgerät gemäß Anspruch 4 und einem der vorangehenden Ansprüche, wobei der erste Schwenkarm (101) ein erstes Gleitelement (101b) aufweist, das so angeordnet ist, dass es während der Schwenkbewegung des Türblattelements (120) zwischen der ersten Position (P1) und der zweiten Position (P2) im Kontakt mit der ersten Rahmenseitenwand (89) an dessen der Kammer (2) abgewandten Außenseite entlang gleitet, und
wobei insbesondere der zweite Schwenkarm (102) ein zweites Gleitelement (102b) aufweist, das so angeordnet ist, dass es während der Schwenkbewegung des Türblattelements (120) zwischen der ersten Position (P1) und der zweiten Position (P2) im Kontakt mit der zweiten Rahmenseitenwand (88) an dessen der Kammer (2) abgewandten Außenseite entlang gleitet.

10. Laborgerät gemäß Anspruch 9, wobei der erste Schwenkarm (101) ein erstes plattenförmiges Bauteil (101a) aufweist, das mit dem ersten Schwenkarm (101) fest verbunden ist und das dieses erste Gleitelement (101b) trägt, und
wobei insbesondere der zweite Schwenkarm (102) ein zweites plattenförmiges Bauteil (102a) aufweist, das mit dem zweiten Schwenkarm (102) fest verbunden ist und das dieses zweite Gleitelement (102b) trägt,
wobei sich das erste plattenförmige Bauteil (101a) insbesondere entlang der Länge des ersten Schwenkarms (101) erstreckt, und wobei sich das zweite plattenförmige Bauteil (102a) insbesondere entlang der Länge des zweiten Schwenkarms (102) erstreckt.

11. Laborgerät gemäß einem der vorangehenden Ansprüche, wobei in einer planaren Frontwand (10) des Laborgeräts im Abstand zu der Kammer (2) eine schlitzförmige Aussparung (10c) vorgesehen ist, die sich senkrecht zu der Frontwand (10) in das Innere des Laborgeräts erstreckt, und in der der mindestens eine erste Schwenkarm (101; 103) in der ersten Position (P1) des Türblattelements (120) angeordnet ist, und wobei insbesondere in der planaren Frontwand (10) des Laborgeräts im Abstand zu der Kammer (2) eine weitere schlitzförmige Aussparung (10d) vorgesehen ist, die sich senkrecht zu der Frontwand (10) in das Innere des Laborgeräts erstreckt, und in der der mindestens eine zweite Schwenkarm (102; 104) in der ersten Position (P1) des Türblattelements (120) angeordnet ist.

12. Laborgerät gemäß einem der vorangehenden Ansprüche, wobei in keiner Position des Türblattelements (120) ein Schwenkarm (101; 102; 103; 104) der Schwenktüreinrichtung (100) teilweise oder vollständig innerhalb der Kammer (2) angeordnet ist.

13. Laborgerät gemäß einem der vorangehenden Ansprüche, wobei das Türblattelement (120) eine Heizeinrichtung aufweist, durch die das Türblattelement (120), insbesondere das Sichtfenster (170), beheizbar ist.

14. Laborgerät gemäß einem der vorangehenden Ansprüche, wobei um die Kammeröffnung herum eine elastomere Dichtung, insbesondere eine Silikondichtung, angeordnet ist, welche in der Position P1 des Türblattelements (120) vomTürblattelement (120) kontaktiert wird.

15. Laborgerät gemäß einem der vorangehenden Ansprüche, das ein Inkubationsschüttler ist.
